# EUROPEAN PATENT SPECIFICATION

(11) **EP 2 579 952 B1**
(45) Date of publication and mention of the grant of the patent: **08.11.2017**
(21) Application number: 11725874.9
(22) Date of filing: 08.06.2011
(51) Int. Cl.: A61Q 19/08, A61K 8/64, A61K 8/99

(54) **SKIN ANTIAGING TREATMENT**
BEHANDLUNG GEGEN HAUTALTERUNG
TRAITEMENT ANTIVIEILLISSEMENT POUR LA PEAU

(30) Priority: 09.06.2010 US 797222
(43) Date of publication of application: 17.04.2013
(73) Proprietor: Lipotec, S.A., 8550 Gava (ES)
(72) Inventor: CEBRIAN PUCHE, Juan, E-08020 Barcelona (ES); ALMIÑANA DOMENECH, Nuria, E-08030 Barcelona (ES); DELGADO GONZALEZ, Raquel, E-08850 Gava-Barcelona (ES)
(74) Representative: Carvajal y Urquijo, Isabel
(86) International application number: PCT/EP2011/002799
(87) International publication number: WO 2011/154126

(56) References cited:
- EP-A1- 1 180 524
- EP-A1- 1 892 247
- EP-A1- 2 123 673
- WO-A1-97/34620
- US-A1- 2007 081 960

SEQUENCE LISTING

<110>  LIPOTEC SA

<120>  SKIN ANTIAGING TREATMENT

<130>  PXW000510/2011

<160>  39

<170>  PatentIn version 3.5

<210>  1
<211>  206
<212>  PRT
<213>  Homo sapiens

<400>  1

<210>  2
<211>  84
<212>  PRT
<213>  Homo sapiens

<400>  2

Met Ala Glu Asp Ala Asp Met Arg Asn Glu Leu Glu Glu Met Gln Arg
1               5                   10                  15

Arg Ala Asp Gln Leu Ala Asp Glu Ser Leu Glu Ser Thr Arg Arg Met
            20                  25                  30

Leu Gln Leu Val Glu Glu Ser Lys Asp Ala Gly Ile Arg Thr Leu Val
        35                  40                  45

Met Leu Asp Glu Gln Gly Glu Gln Leu Glu Arg Ile Glu Glu Gly Met
    50                  55                  60

Asp Gln Ile Asn Lys Asp Met Lys Glu Ala Glu Lys Asn Leu Thr Asp
65                  70                  75                  80

Leu Gly Lys Phe

<210>  3
<211>  37
<212>  PRT
<213>  Homo sapiens

<400>  3

Glu Ile Asp Thr Gln Asn Arg Gln Ile Asp Arg Ile Met Glu Lys Ala
1               5                   10                  15

Asp Ser Asn Lys Thr Arg Ile Asp Glu Ala Asn Gln Arg Ala Thr Lys
            20                  25                  30

Met Leu Gly Ser Gly
        35

<210>  4
<211>  13
<212>  PRT
<213>  Homo sapiens

<400>  4

Glu Leu Glu Glu Met Gln Arg Arg Ala Asp Gln Leu Ala
1               5                   10

<210>  5
<211>  16
<212>  PRT
<213>  Homo sapiens

<400>  5

Ser Leu Glu Ser Thr Arg Arg Met Leu Gln Leu Val Glu Glu Ser Lys
1               5                   10                  15

<210>  6
<211>  17
<212>  PRT
<213>  Homo sapiens

<400>  6

Asp Gln Ile Asn Lys Asp Met Lys Glu Ala Glu Lys Asn Leu Thr Asp
1               5                   10                  15

Leu

<210>  7
<211>  26
<212>  PRT
<213>  Homo sapiens

<400>  7

Ile Met Glu Lys Ala Asp Ser Asn Lys Thr Arg Ile Asp Glu Ala Asn
1               5                   10                  15

Gln Arg Ala Thr Lys Met Leu Gly Ser Gly
            20                  25

<210>  8
<211>  8
<212>  PRT
<213>  Homo sapiens

<400>  8

Glu Glu Met Gln Arg Arg Ala Asp
1               5

<210>  9
<211>  13
<212>  PRT
<213>  Homo sapiens

<400>  9

Leu Glu Ser Thr Arg Arg Met Leu Gln Leu Val Glu Glu
1               5                   10

<210>  10
<211>  12
<212>  PRT
<213>  Homo sapiens

<400>  10

Asn Lys Asp Met Lys Glu Ala Glu Lys Asn Leu Thr
1               5                   10

<210>  11
<211>  6
<212>  PRT
<213>  Homo sapiens

<400>  11

Glu Glu Met Gln Arg Arg
1               5


<210>  12
<211>  21
<212>  PRT
<213>  Homo sapiens

<400>  12

Met Ala Glu Asp Ala Asp Met Arg Asn Glu Leu Glu Glu Met Gln Arg
1               5                   10                  15


Arg Ala Asp Gln Leu
            20


<210>  13
<211>  23
<212>  PRT
<213>  Homo sapiens

<400>  13

Ala Asp Glu Ser Leu Glu Ser Thr Arg Arg Met Leu Gln Leu Val Glu
1               5                   10                  15


Glu Ser Lys Asp Ala Gly Ile
            20


<210>  14
<211>  12
<212>  PRT
<213>  Homo sapiens

<400>  14

Glu Leu Glu Glu Met Gln Arg Arg Ala Asp Gln Leu
1               5                   10


<210>  15
<211>  11
<212>  PRT
<213>  Homo sapiens

<400>  15

Glu Leu Glu Glu Met Gln Arg Arg Ala Asp Gln
1               5                   10


<210>  16
<211>  10
<212>  PRT
<213>  Homo sapiens

<400>  16

Glu Leu Glu Glu Met Gln Arg Arg Ala Asp
1               5                   10


<210>  17
<211>  9
<212>  PRT
<213>  Homo sapiens

<400>  17

Glu Leu Glu Glu Met Gln Arg Arg Ala
1               5


<210>  18
<211>  8
<212>  PRT
<213>  Homo sapiens

<400>  18

Glu Leu Glu Glu Met Gln Arg Arg
1               5


<210>  19
<211>  11
<212>  PRT
<213>  Homo sapiens

<400>  19

Leu Glu Glu Met Gln Arg Arg Ala Asp Gln Leu
1               5                   10


<210>  20
<211>  10
<212>  PRT
<213>  Homo sapiens

<400>  20

Leu Glu Glu Met Gln Arg Arg Ala Asp Gln
1               5                   10


<210>  21
<211>  9
<212>  PRT
<213>  Homo sapiens

<400>  21

Leu Glu Glu Met Gln Arg Arg Ala Asp
1               5


<210>  22
<211>  8
<212>  PRT
<213>  Homo sapiens

<400>  22

Leu Glu Glu Met Gln Arg Arg Ala

1                   5


<210>  23
<211>  7
<212>  PRT
<213>  Homo sapiens

<400>  23

Leu Glu Glu Met Gln Arg Arg
1               5


<210>  24
<211>  10
<212>  PRT
<213>  Homo sapiens

<400>  24

Glu Glu Met Gln Arg Arg Ala Asp Gln Leu
1               5                   10


<210>  25
<211>  9
<212>  PRT
<213>  Homo sapiens

<400>  25

Glu Glu Met Gln Arg Arg Ala Asp Gln
1               5


<210>  26
<211>  7
<212>  PRT
<213>  Homo sapiens

<400>  26

Glu Glu Met Gln Arg Arg Ala
1               5


<210>  27
<211>  6
<212>  PRT
<213>  Homo sapiens

<400>  27

Lys Asn Leu Thr Asp Leu
1               5


<210>  28
<211>  20
<212>  PRT
<213>  Homo sapiens

<400>  28

Ser Asn Lys Thr Arg Ile Asp Glu Ala Asn Gln Arg Ala Thr Lys Met
1               5                   10                  15

Leu Gly Ser Gly
            20


<210>  29
<211>  17
<212>  PRT
<213>  Homo sapiens

<400>  29

Thr Arg Ile Asp Glu Ala Asn Gln Arg Ala Thr Lys Met Leu Gly Ser
1               5                   10                  15


Gly



<210>  30
<211>  14
<212>  PRT
<213>  Homo sapiens

<400>  30

Asp Glu Ala Asn Gln Arg Ala Thr Lys Met Leu Gly Ser Gly
1               5                   10


<210>  31
<211>  11
<212>  PRT
<213>  Homo sapiens

<400>  31

Asn Gln Arg Ala Thr Lys Met Leu Gly Ser Gly
1               5                   10


<210>  32
<211>  10
<212>  PRT
<213>  Homo sapiens

<400>  32

Gln Arg Ala Thr Lys Met Leu Gly Ser Gly
1               5                   10


<210>  33
<211>  5
<212>  PRT
<213>  Homo sapiens

<400>  33

Tyr Gly Gly Phe Leu
1               5


<210>  34
<211>  5
<212>  PRT
<213>  Homo sapiens

<400>  34

Tyr Gly Gly Phe Met
1               5

<210>  35
<211>  5
<212>  PRT
<213>  Homo sapiens

<400>  35

Tyr Ala Gly Phe Leu
1               5

<210>  36
<211>  5
<212>  PRT
<213>  Homo sapiens

<400>  36

Tyr Ser Gly Phe Leu
1               5

<210>  37
<211>  5
<212>  PRT
<213>  Homo sapiens

<400>  37

Tyr Ala Gly Phe Met
1               5

<210>  38
<211>  5
<212>  PRT
<213>  Homo sapiens

<400>  38

Tyr Ser Gly Phe Met
1               5

<210>  39
<211>  6
<212>  PRT
<213>  Homo sapiens

<400>  39

Glu Asp Tyr Tyr Arg Leu
1               5

## Description

### FIELD OF THE INVENTION

The present invention refers to a method for skin antiaging treatment comprising administering Botulinum toxin to an area of facial and/or neck skin, combined with the administration of a cosmetic composition comprising a cosmetically effective amount of at least one peptide derived from the SNAP-25 protein and/or at least one enkephalin-derived peptide, and at least one cosmetically acceptable excipient or adjuvant. The combined treatment prolongs in time the antiaging effectiveness of Botulinum toxin injections.

### BACKGROUND OF THE INVENTION

Expression wrinkles are the wrinkles resulting from the stress exerted by the contractions of facial muscles responsible for causing facial expressions on the skin of the face. Expression wrinkles are usually located on the forehead, in the space between the eyebrows, around the mouth and/or around the eyes. Depending on the shape of the face, the expression frequency and the existence of tics (convulsive movements which are frequently repeated, caused by the involuntary contraction of one or several muscles, in this case facial muscles), expression wrinkles may even appear during adolescence. External factors such as exposure to the sun emphasize their depth and visibility.

The basis or mechanism for the formation of these facial wrinkles is the tensing of the muscles of the epidermis that drag the skin inwards. This muscular tension is the result of hyperactivity of the nerves innervating the facial muscles. Nerve hyperactivity is characterized by the uncontrolled and excessive release of neurotransmitters that excite muscle fibers. Because of this, the molecules that control neuronal exocytosis contribute to relaxing muscular tension, and consequently, to eliminating wrinkles.

Botulinum toxin injections have been widely used for such purposes, in particular the A serotype (BOTOX® Cosmetic/Vistabel, Allergan Inc., Dysport™, Ipsen Biopharm, Ltd. and Azzalure®, Galderma S.A.) *>**B*Carruthers, A., Kiene, K. and Carruthers, J. (1996) "Botulinum A exotoxin use in clinical dermatology" J. Am. Acad. Dermatol. 34, 788-797*;* Cheng, C.M. (2007) "Cosmetic use of botulinum toxin type A in the elderly" Clin. Interv. Aging. 2, 81-83*;* Ascher, B, Talarico, S., Cassuto, D., Escobar, S., Hexsel, D., Jaén, P., Monheit, G., Rzany, B., Viel, M. (2010) "International consensus recommendations on the aesthetic usage of botulinum toxin type A (Speywood Unit) - part I: upper facial wrinkles" J. Eur. Acad. Dermatol. Venereol.*].* Such techniques deliver toxin into muscles to produce a local effect. Botulinum neurotoxins inhibit the neuronal exocytosis in the neuromuscular junction (nerve-muscle synapse) by cleaving any of the proteins that make up the SNARE complex which directs and controls the release of acetylcholine accumulated in vesicles, thus causing weakness of the nearby muscle.

The paralytic effects of the toxin are reversible with an average duration of 3-4 months, depending on the severity of wrinkles and the strength of muscles treated *[*Rzany, B., Ascher, B., Monheit, G.D. (2010) "Treatment of glabellar lines with botulinum toxin type A (Speywood Unit): a clinical overview" J. Eur. Acad. Dermatol. Venereol. 24, S1, 1-14*]*. The maximum of efficacy after Botulinum toxin injection is obtained during the first month after the administration, with a sharp decay along the next couple of months, i.e. 2 months after Botulinum toxin injection the wrinkle reduction average is ca. 40% of the maximum of the wrinkle reduction average, and ca. 20% 4 months after the injection. The treatment therefore requires the repeated injection of Botulinum toxin, which may trigger an immune response which will end causing a clear loss of treatment efficacy. This loss of treatment efficacy with Botulinum toxin entails the need to increase the administered dose in subsequent treatments, which in turn causes a potentiation of the immune response. As an alternative to the treatment with Botulinum toxin serotype A, the use of different serotypes of Botulinum toxins, such as serotype B, serotype F and serotype E, has been considered. Nevertheless, the application of different serotypes cannot be considered a solution to the problem, because sooner or later, the immune reaction can occur again. Furthermore, the treatment with Botulinum toxins for aesthetic improvement is not exempt from adverse side effects, being the most frequently reported (≥10% prevalence) injection site reactions and headache, but also with a non negligible prevalence (≥1-<10%) ptosis, facial paresis, lacrimation increase or dry eye, eyelid oedema, asthenopia and muscle twitching around eyes. Such side effects are transient and moderate in intensity but could last several weeks, and may discourage volunteers to reinject the toxin as soon as the wrinkles reappear. Botulinum toxin reinjection frequency must be consensuated with the physician who must balance between the benefit and the potential treatment-emergent side effects. As a regular medical praxis, physicians recommend reinjections every 6 months.

The cosmetic industry has made a lot of efforts to develop new molecules that imitate the paralytic effects of the Botulinum toxins, but with much simpler and more stable molecular structures, which do not induce immune responses, and whose manufacturing cost is economical. Peptide-type molecules comply with these properties and solve the problems presented by treatment with Botulinum toxin. Specifically, the patent EP 1180524 B1 and the patent application WO 2008/049945 A1 of Lipotec, S.A. describe peptides derived from the amino terminal fragment of the SNAP-25 protein which inhibit neuronal exocytosis and thus possess anti-wrinkle effect. The international application WO 97/34620 A1 also describes peptides derived from the amino acid sequence of the SNAP-25 protein, in particular its carboxy-terminal, or of synaptobrevin or syntaxin capable of inhibiting neuronal exocytosis.

A different approach for inhibiting muscle contraction is that achieved by acting at the post-synaptic level. This is the approach of some commercially available anti-expression wrinkle peptides such us Inyline™ [INCI: Acetyl Hexapeptide-30] marketed by Lipotec which interferes with the clustering of the acetylcholine receptor, or Vialox® [INCI: Pentapeptide-3] and Syn®-Ake [INCI: Dipeptide Diaminobutyroyl Benzylamide Diacetate], marketed by Pentapharm/DSM, which are antagonists of the acetylcholine receptor.

Other methods described for the reduction and/or elimination of expression wrinkles involve the use of calcium channel antagonists, particularly salts of manganese (FR 2809005 A1), alverine (FR 2798590 A1) or magnesium gluconate (FR 2846885 A1), or the use of chloride channel agonists such as glycine (EP 0704210 A2) or Iris pallida extracts (FR 2746641 A1).

A different mechanism of action for inhibiting neuronal exocytosis is described in the patent application EP 1892247 A1 of Lipotec, S.A. The document describes a family of peptides derived from the enkephalin sequence which are effective in the reduction and/or elimination of facial wrinkles, especially expression wrinkles, acting by activating K⁺ currents in neurons causing a hyperpolarizing effect which results in a reduction in Ca²⁺-dependent neuronal exocytosis.

In patent application EP 1892247 A1 of Lipotec, S.A. it is also described the effect of the combination of SNAP-25 derived peptides with enkephalin-derived peptides. The combination of both complementary mechanisms to attenuate muscle contraction provides an enhanced and sustained efficacy in reducing expression wrinkles.

There remains the need for improvement in performing Botulinum toxin treatments, and it is especially necessary to prolong the benefits received from the treatment and/or minimize the need for multiple treatments for a cosmetically noticeable outcome.

Surprisingly, the inventors have found that the administration in areas of facial and/or neck skin of a cosmetic composition comprising peptides derived from the SNAP-25 protein, which block neuronal exocytosis, and/or enkephalin-derived peptides, which reduce Ca²⁺-dependent neuronal exocytosis, prolong in time the antiaging effectiveness of Botulinum toxin injections.

### DESCRIPTION OF THE INVENTION

This invention provides a solution to the above mentioned needs. This invention provides a method for skin antiaging treatment comprising administering Botulinum toxin to an area of facial and/or neck skin, combined with the administration of a cosmetic composition comprising a cosmetically effective amount of at least one peptide derived from the SNAP-25 protein, and/or at least one enkephalin-derived peptide, and at least one cosmetically acceptable excipient or adjuvant. The skin antiaging treatment is a treatment for reducing or eliminating wrinkles of the facial and/or neck skin.

This invention provides a simple, effective and risk-free solution for prolonging in time the temporary effects of the treatment with Botulinum toxin injections. The administration of a cosmetic composition containing peptides derived from the SNAP-25 protein and/or enkephalin-derived peptides maintains the benefit of the Botulinum toxin administration beyond the usual average of three-four months, and allows reducing the frequency of Botulinum toxin injections to two or less times per year. The anti-wrinkle effect of Botulinum toxin injections is better mantained until the next Botulinum toxin administration, and also the next Botulinum toxin administration can be delayed, reducing this way the probability of an immune response of the patient and loss of effectiveness of the treatment as a consequence of repeated administrations of Botulinum toxin. The treatment of this invention provides a wrinkle reduction average higher than 50% after 2 months in relation to the maximum of the wrinkle reduction average, higher than 25% after 4 months and higher than 10% after 6 months.

### Definitions

In order to facilitate the comprehension of this invention, the meanings of some terms and expressions as they are used within the context of the invention are included.

Within the context of this invention the terms "aging" and "skin aging" are used to describe the emergence of visible changes in skin appearance as well as those perceptible by touch, such as for example and in a non-limiting sense wrinkles, fine lines, roughness, expression lines, stretch marks, discontinuities, furrows, flaccidity, skin sagging, such as cheeks sagging, eye pouches, double chin, increase of pore size, loss of elasticity, loss of resilience, loss of firmness, elastosis, anomalous differentiation, hyperkeratinization, keratosis, changes of the skin color, such as marks, redness or bags under the eyes, formation of hyperpigmented areas such as age spots, melasma or freckles, loss of smoothness, orange-peel skin, loss of collagen structure and other histological changes of the stratum corneum, of the dermis, epidermis, vascular system (for example the formation of spider veins or telangiectasias) or of those tissues close to the skin. Skin aging is a process with two main components: chronological, which is due to the passage of time, and photo-induced, which is due to the level of exposure to ultraviolet (UV) radiation and which is known as photoaging. The sum of several environmental factors such as exposure to tobacco smoke, exposure to pollution, and climatic conditions like cold and/or wind also contribute to skin aging.

Within the context of this invention, "skin antiaging treatment" is a treatment for preventing, delaying and/or reducing the aging of the skin in humans.

In the present invention, the abbreviations used for amino acids follow the rules of the IUPAC-IUB Joint Commission on Biochemical Nomenclature specified in Eur. J. Biochem., 1984, 138:9-37 and in J. Biol. Chem., 1989, 264:633-673. Thus, for example, Gly represents NH₂-CH₂-CO-OH, Gly- represents NH₂-CH₂-CO-, -Gly represents -NH-CH₂-CO-OH and -Gly- represents -NH-CH₂-CO-. The hyphen, representing the peptide bond, therefore eliminates the OH from the 1-carboxyl group of the amino acid (represented herein in the conventional non-ionized form) when it is placed to the right of the symbol, and eliminates the H from the 2-amino group of the amino acid when it is placed to the left of the symbol; both modifications can be applied to the same symbol.

The abbreviation "Ac-" is used in the present description to designate the acetyl (CH₃-CO-) group and the abbreviation "Palm-" is used to designate the palmitoyl (CH₃-(CH₂)₁₄-CO-) group.

Within the context of this invention, the term "non-cyclic aliphatic group" is used in this invention to encompass, for example and not limited thereto, alkyl, alkenyl and alkynyl groups, linear or branched.

The term "alkyl group" refers in this invention to a linear or branched saturated group, having 1 to 24, preferably 1 to 16, even more preferably 1 to 14, still more preferably 1 to 12, still more preferably 1, 2, 3, 4, 5 or 6 carbon atoms, and which is bound to the rest of the molecule through a simple bond, including, for example and not limited thereto, methyl, ethyl, isopropyl, isobutyl, *tert*-butyl, heptyl, octyl, decyl, dodecyl, lauryl, hexadecyl, octadecyl, amyl, 2-ethylhexyl, 2-methylbutyl, 5-methylhexyl and the like.

The term "alkenyl group" refers in this invention to a linear or branched unsaturated group that has between 2 and 24, preferably between 2 and 16, even more preferably between 2 and 14, still more preferably between 2 and 12, still more preferably 2, 3, 4, 5 or 6 carbon atoms with one or more carbon-carbon double bonds, preferably with 1, 2 or 3 carbon-carbon double bonds, conjugated or not conjugated, which is bound to the rest of the molecule through a simple bond, including for example and not limited thereto, the vinyl, oleyl, linoleyl group and the like.

The term "alkynyl group" refers in this invention to a linear or branched unsaturated group that has between 2 and 24, preferably between 2 and 16, even more preferably between 2 and 14, still more preferably between 2 and 12, still more preferably 2, 3, 4, 5 or 6 carbon atoms with one or more triple carbon-carbon bonds, preferably 1, 2 or 3 triple carbon-carbon bonds, conjugated or not conjugated, which is bound to the rest of the molecule through a simple bond, including, for example and not limited thereto, the ethynyl group, 1-propynyl, 2-propynyl, 1-butynyl, 2-butynyl, 3-butinyl, pentynyl, e.g. 1-pentynyl, and the like.

The term "alicyclyl group" is used in this invention to encompass, for example, and not limited thereto, cycloalkyl or cycloalkenyl or cycloalkynyl groups.

The term "cycloalkyl" refers in this invention to a mono- or polycyclic saturated aliphatic group which has between 3 and 24, preferably between 3 and 16, even more preferably between 3 and 14, still more preferably between 3 and 12, and still more preferably 3, 4, 5 or 6 carbon atoms and which is bound to the rest of the molecule through a simple bond, including, for example and not limited thereto, cyclopropyl, cyclobutyl, cyclopentyl, cyclohexyl, cycloheptyl, methyl cyclohexyl, dimethyl cyclohexyl, octahydroindene, decahydronaphtalene, dodecahydro phenalene and the like.

The term "cycloalkenyl" refers in this invention to a mono- or polycyclic non-aromatic aliphatic group that has between 5 and 24, preferably between 5 and 16, even more preferably between 5 and 14, still more preferably between 5 and 12, and still more preferably 5 or 6 carbon atoms with one or more carbon-carbon double bonds, preferably 1, 2 or 3 carbon-carbon double bonds, conjugated or not conjugated, and which is bound to the rest of the molecule through a simple bond, including for example and not limited thereto, the cyclopent-1-en-1-yl group and the like.

The term "cycloalkynyl" refers in this invention to a mono- or polycyclic non-aromatic aliphatic group that has between 8 and 24, preferably between 8 and 16, even more preferably between 8 and 14, still more preferably between 8 and 12, and still more preferably 8 or 9 carbon atoms with one or more carbon-carbon triple bonds, preferably 1, 2 or 3 carbon-carbon triple bonds, conjugated or not conjugated, and which is bound to the rest of the molecule through a simple bond, including for example and not limited thereto, the cyclooct-4-en-2-ynyl group and the like.

The term "aryl group" refers in this invention to an aromatic group which has between 6 to 30, preferably between 6 and 18, even more preferably between 6 and 10, and still more preferably 6 or 10 carbon atoms, composed of 1 2, 3 or 4 aromatic rings linked through a carbon-carbon bond or fused, including, for example and not limited thereto, phenyl, naphthyl, diphenyl, indenyl, phenanthryl or anthranyl, among others, or an aralkyl group.

The term "aralkyl group" refers in this invention to an alkyl group substituted with an aromatic group, having between 7 and 24 carbon atoms, which is bound to the rest of the molecule through a simple bond, and including, for example and not limited thereto, -(CH₂)₁₋₆-phenyl, -(CH₂)₁₋₆-(1-naphthyl), -(CH₂)₁₋₆-(2-naphthyl), -(CH₂)₁₋₆-CH(phenyl)₂ and the like.

The term "heterocyclyl group" refers in this invention to a hydrocarbon ring with 3-10 members in which one or more of the ring atoms, preferably 1, 2 or 3 ring atoms is an element different from carbon such as for example nitrogen, oxygen or sulfur and which may be saturated or unsaturated. For the purposes of this invention, the heterocycle can be a cyclic, mono-cyclic, bicyclic or tricyclic system, which may include fused ring systems, and atoms of nitrogen, carbon or sulfur may optionally be oxidized in the heterocyclyl radical; the nitrogen atom can be optionally quaternized and the radical heterocyclyl can be partially or completely saturated or be aromatic. More preferably, the term heterocyclyl refers to a ring with 5 or 6 members. Examples of saturated heterocyclyl groups are dioxane, piperidine, piperazine, pyrrolidine, morpholine and thiomorpholine. Examples of aromatic heterocyclyl groups, also known as heteroaromatic groups, are pyridine, pyrrole, furan, thiophene, benzofuran, imidazoline, quinoleine, quinoline, pyridazine and naphthyridine.

The term "heteroarylalkyl group" refers in this invention to an alkyl group substituted with a substituted or non-substituted aromatic heterocyclyl group, the alkyl group having 1 to 3 carbon atoms and the aromatic heterocyclyl group between 2 and 24 carbon atoms and from 1 to 3 atoms other than carbon, which is bound to the rest of the molecule through a simple bond, and including, for example and not limited thereto, -(CH₂)₁₋₆-imidazolyl, -(CH₂)₁₋₆-triazolyl, -(CH₂)₁₋₆-thienyl, -(CH₂)₁₋₆-furyl-(CH₂)₁₋₆-pyrrolidinyl and the like.

As it is understood in this technical area, there may be a certain degree of substitution in the previously defined groups. Thus, there may be a substitution in any of the groups of this invention. References in this document to substituted groups in the groups of this invention indicate that the specified radical may be substituted in one or more available positions by one or more substituents, preferably in 1, 2 or 3 positions, more preferably in 1 or 2 positions, and even more preferably in 1 position. Such substituents include, for example, and not limited thereto, C₁-C₄ alkyl; hydroxyl; C₁-C₄ alkoxyl; amino; C₁-C₄ aminoalkyl; C₁-C₄ carbonyloxyl; C₁-C₄ oxycarbonyl; halogen such as fluorine, chlorine, bromine and iodine; cyano; nitro; azido; C₁-C₄ alkylsulfonyl; thiol; C₁-C₄ alkylthio; aryloxyl such as phenoxyl, -NR_{b}(C=NR_{b})NR_{b}R_{c}; wherein R_{b} and R_{c} are independently selected from the group consisting of H, C₁-C₄ alkyl, C₂-C₄ alkenyl, C₂-C₄ alkynyl, C₃-C₁₀ cycloalkyl, C₆-C₁₈ aryl, C₇-C₁₇ aralkyl, heterocyclic with 3-10 members or amino protective group.

### Method of the invention

In a first aspect, this invention refers to a method for skin antiaging treatment that prolongs in time the effects of treatment with Botulinum toxin injections, comprising:
a. The administration of an effective amount of Botulinum toxin to an area of facial and/or neck skin,
b. and the administration, from once a week to ten times a day, of a cosmetic composition comprising a cosmetically effective amount of at least one peptide according to claim 1 which contains a sequence of 6 to 40 adjacent amino acids contained in the amino acid sequence of the SNAP-25 protein, defined by SEQ ID No.1, and according to the general formula (I):

   R₁-AA-R₂ (I)

   its stereoisomers, mixtures thereof, and/or its cosmetically acceptable salts thereof, in which AA is a sequence of 6 to 40 adjacent amino acids contained in the amino acid sequence SEQ ID No.1;
   and/or at least one enkephalin-derived peptide of general formula (II): 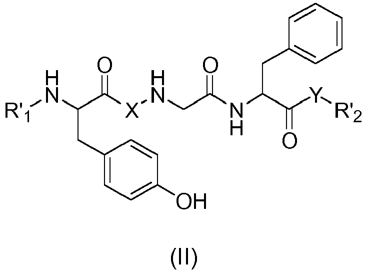
   its stereoisomers, mixtures thereof, and/or its cosmetically acceptable salts thereof,
   wherein:
   X and Y are independently selected from the group consisting of natural amino acids and non-natural amino acids;
   R₁ and R'₁ are independently selected from the group consisting of H, substituted or non-substituted non-cyclic aliphatic group, substituted or non-substituted alicyclyl, substituted or non-substituted heterocyclyl, substituted or non-substituted heteroarylalkyl, substituted or non-substituted aryl, substituted or non-substituted aralkyl and R₅-C(O)-; and
   R₂ and R'₂ are independently selected from the group consisting of -NR₃R₄, -OR₃ and -SR₃; where R₃ and R₄ are independently selected from the group consisting of H, substituted or non-substituted non-cyclic aliphatic group, substituted or non-substituted alicyclyl, substituted or non-substituted heterocyclyl, substituted or non-substituted heteroarylalkyl, substituted or non-substituted aryl and substituted or non-substituted aralkyl;
   wherein R₅ is selected from the group consisting of H, substituted or non-substituted non-cyclic aliphatic group, substituted or non-substituted alicyclyl, substituted or non-substituted aryl, substituted or non-substituted aralkyl, substituted or non-substituted heterocyclyl and substituted or non-substituted heteroarylalkyl;
   and at least one cosmetically acceptable excipient or adjuvant.

The R₁, R'₁ and R₂, R'₂ groups are bound at the amino-terminal (*N*-terminal) and carboxy-terminal (*C*-terminal) ends of the peptide sequences, respectively.

In one particular embodiment, the skin antiaging treatment is a treatment for reducing or eliminating wrinkles of the facial and/or neck skin, preferably expression wrinkles.

In another particular embodiment, the preferred structures of the peptides represented in the general formula (II) are those where X is -Gly-, -Ala- or -Ser-, and more preferably -D-Ala- or -D-Ser-.

In another particular embodiment, the preferred structures of the peptides represented in the general formula (II) are those where Y is -Leu- or -Met-, and more preferably - L-Leu- or -L-Met-.

In another particular embodiment, R₁ and R'₁ are independently selected from the group consisting of H, a polymer of general formula (III) 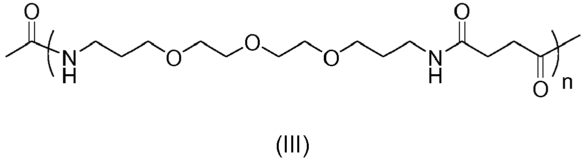 wherein n ranges between 1 to 100, preferably between 1 and 5, and R₅-CO-, wherein R₅ is selected from the group consisting of substituted or unsubstituted C₁-C₂₄ alkyl radical, substituted or unsubstituted C₂-C₂₄ alkenyl radical, substituted or unsubstituted C₂-C₂₄ alkynyl radical, substituted or unsubstituted C₃-C₂₄ cycloalkyl radical, substituted or unsubstituted C₅-C₂₄ cycloalkenyl radical, substituted or unsubstituted C₈-C₂₄ cycloalkynyl radical, substituted or unsubstituted C₆-C₃₀ aryl radical, substituted or unsubstituted C₇-C₂₄ aralkyl radical, a substituted or unsubstituted heterocyclyl radical having 3-10 ring members, a substituted or unsubstituted heteroarylalkyl radical having 2 to 24 carbon atoms and having 1 to 3 atoms other than carbon wherein the alkyl chain is of 1 to 6 carbon atoms. Preferably, R₁ and R'₁ are independently selected from the group consisting of H, acetyl, *tert*-butanoyl, hexanoyl, 2-methylhexanoyl, cyclohexanecarboxyl, octanoyl, decanoyl, lauroyl, myristoyl, palmitoyl, stearoyl, behenyl, oleoyl and linoleoyl. More preferably, R₁ and R'₁ are independently selected from the group consisting of H, acetyl, hexanoyl, octanoyl, lauroyl, myristoyl or palmitoyl.

In another particular embodiment, R₂ and R'₂ are independently selected from the group consisting of -NR₃R₄, -OR₃ or -SR₃ where R₃ and R₄ are independently selected from the group consisting of H, substituted or unsubstituted C₁-C₂₄ alkyl, substituted or unsubstituted C₂-C₂₄ alkenyl, substituted or unsubstituted C₂-C₂₄ alkynyl, substituted or unsubstituted C₃-C₂₄ cycloalkyl, substituted or unsubstituted C₅-C₂₄ cycloalkenyl, substituted or unsubstituted C₈-C₂₄ cycloalkynyl, substituted or unsubstituted C₆-C₃₀ aryl, substituted or unsubstituted C₇-C₂₄ aralkyl, a substituted or unsubstituted heterocyclyl having 3-10 ring members, and a substituted or unsubstituted heteroarylalkyl group having 2 to 24 carbon atoms and having 1 to 3 atoms other than carbon wherein the alkyl chain is of 1 to 6 carbon atoms and a polymer of general formula (III) wherein n ranges between 1 and 100, preferably between 1 and 5. Optionally, R₃ and R₄ can be bound by means of a saturated or unsaturated carbon-carbon bond, forming a cycle with the nitrogen atom. Preferably, R₂ and R'₂ are independently selected from the group consisting of -NR₃R₄ or -OR₃, wherein R₃ and R₄ are independently selected from the group consisting of H, substituted or unsubstituted C₁-C₂₄ alkyl, substituted or unsubstituted C₂-C₂₄ alkenyl, substituted or unsubstituted C₂-C₂₄ alkynyl, substituted or unsubstituted C₃-C₁₀ cycloalkyl, substituted or unsubstituted C₆-C₁₅ aryl and a substituted or unsubstituted heterocyclyl of 3-10 members, a substituted or unsubstituted heteroarylalkyl group with a ring having 3 to 10 members and an alkyl chain of 1 to 6 carbon atoms and a polymer of general formula (III) wherein n ranges between between 1 and 100, preferably between 1 and 5. More preferably, R₃ and R₄ are selected from the group consisting of H, methyl, ethyl, hexyl, dodecyl and hexadecyl. Even more preferably, R₃ is H and R₄ is selected from the group consisting of H, methyl, ethyl, hexyl, dodecyl and hexadecyl. According to an even more preferred embodiment, R₂ and R'₂ are independently selected from the group consisting of -OH and -NH₂.

Peptides comprised in the cosmetic composition of the method of this invention may exist as stereoisomers or mixtures of stereoisomers; for example, the amino acids that make them up can have L-, D-configuration or be racemic independently from each other. Therefore, it is possible to obtain isomeric mixtures as well as racemates or diastereomeric mixtures or pure diastereomers or enantiomers, depending on the number of asymmetric carbons and what isomers or isomeric mixtures are present.

The preferred structures of the peptides comprised in the cosmetic composition of the method of this invention are pure isomers, i.e., enantiomers or diastereomers. For example, unless otherwise indicated, it is understood that when it is indicated that one amino acid can be -Ala-, it is understood that it is selected from -L-Ala-, -D-Ala- or racemic or non-racemic mixtures of both. The methods described herein allow the person skilled in the art to obtain each of the stereoisomers of the peptide derivative of the invention by means of choosing the amino acid with the suitable configuration.

In the context of this invention "amino acid sequence derived from the amino acid sequence of the SNAP-25 protein" means any amino acid sequence or fragments of the amino acid sequence of the SNAP-25 protein, defined by the SEQ ID No.1, or any amino acid sequence that differs from the sequence SEQ ID No.1 by mutation, insertion, deletion or substitution of at least one amino acid, or by degeneration of the genetic code, provided that it corresponds to a peptide that possesses the activity of the SNAP-25 protein.

Mutations, insertions or substitutions may take place by genetically encoded amino acids or non-encoded amino acids, natural or not, for example, and not limited thereto, citrulline, ornithine, sarcosine, desmosine, norvaline, 4-aminobutyric acid, 2-aminobutyric acid, 2-aminoisobutyric acid, 6-aminohexanoic acid, 1-naphtylalanine, 2-naphtylalanine, 2-aminobenzoic acid, 4-aminobenzoic acid, 4-chlorophenylalanine, 2,3-diaminopropionic acid, 2,4-diaminobutyric acid, cycloserine, carnitine, cystine, penicillamine, pyroglutamic acid, thienylalanine, hydroxyproline, allo-isoleucine, allo-threonine, isonipecotic acid, isoserine, phenylglycine, statin, beta-alanine, norleucine, *N*-methylamino acids, beta- or gamma-amino acids, among others, and their derivatives. A list of non-natural amino acids can be found in the article "Unusual amino acids in peptide synthesis" by Roberts D.C. and Vellaccio F., in "The Peptides", Vol 5 (1983), Chapter VI, Gross, E. and Meienhofer, J., Eds., Academic Press, New York, USA or in the commercial catalogs of companies specialized in the sector, such as NeoMPS, Bachem, Novabiochem, Sigma-Aldrich, Peptides International, Advanced ChemTech, Chem-Impex, Maybridge Chemical, Chirotech Technology, Peninsula Laboratories or RSP Amino Acid Analogues, among others.

Among the peptides derived from the amino acid sequence of SNAP-25 defined by SEQ ID No.1 included in the cosmetic composition of the method of this invention, are those that possess a sequence of adjacent amino acids contained in the sequence of the carboxy-terminal region of the SNAP-25 protein more preferably in the region between residues 10 to 22, defined by SEQ ID No.4, or specifically contained in the region between residues 12 to 17, defined by SEQ ID No.11, and a sequence of 7 to 12 adjacent amino acids contained in SEQ ID No.4, wherein said sequence comprises the amino acid sequence of SEQ ID No.11.

Furthermore, the cosmetic composition of the method of this invention also comprises peptides substantially homologous to the peptides derived from the amino acid sequence of the SNAP-25 protein, irreversibly chemically modified. "Substantially homologous peptides" means in this invention those amino acid sequences that are at least 60%, preferably 80% and more preferably 95% identical to any of the preceding sequences. The "percentage of identity" refers to the percentage of amino acids that are identical between two compared amino acid sequences, after an optimal alignment of these sequences, where this percentage is purely statistical and differences between the two amino acid sequences are randomly distributed along the sequence. The term "optimal alignment" means the alignment of the amino acid sequences resulting in a higher percentage of identity. The percentage of identity is calculated by determining the number of identical positions where an amino acid is identical in the two sequences compared, dividing the number of identical positions by the number of positions compared and multiplying the result by 100 to get the percentage of identity between the two sequences. Sequence comparisons between two amino acid sequences can be carried out manually or by software such as BLAST algorithm (Basic Local Alignment Search Tool), available online at the site http://blast.ncbi.nlm.nih.gov/.

In the context of this invention "enkephalin-derived peptides" means pentapeptides of general formula (II) with amino acid sequences that differ in 0, 1 or 2 amino acids from the amino acid sequences of the enkephalin pentapeptides defined by the sequences SEQ ID No.33 or SEQ ID No.34.

Among the peptides defined by the general formula (II) included in the cosmetic composition of the method of this invention, preferred amino acid sequences are preferably those sequences selected from the group consisting of SEQ ID No.33, SEQ ID No.34, SEQ ID No.35, SEQ ID No.36, SEQ ID No.37 and SEQ ID No.38. More preferably, amino acid sequences are those sequences selected from the group consisting of SEQ ID No.33 and SEQ ID No.35.

The cosmetic composition of the method of the invention can further comprise a cosmetically effective amount of at least one peptide of general formula (IV):

R"₁-Aₚ-Bᵣ-AA₁-AA₂-AA₃-AA₄-AA₅-AA₆-Cₛ-Dₜ-R"₂ (IV)

its stereoisomers, mixtures thereof, and/or its cosmetically acceptable salts thereof, wherein:
AA₁ is selected from the group consisting of -Asp-, -Glu- and -Pro-;
AA₂ is -Asp-;
AA₃ is selected from the group consisting of -Tyr- and -Arg-;
AA₄ is selected from the group consisting of -Phe- and -Tyr-;
AA₅ is selected from the group consisting of -Arg- and -Lys-;
AA₆ is selected from the group consisting of -Leu- and -Met-;
A, B, C and D are independently selected from the group consisting of natural amino acids and non-natural amino acids;
p, r, s and t are independently selected and range between 0 and 1;
R"₁ is selected from the group consisting of H, substituted or non-substituted non-cyclic aliphatic group, substituted or non-substituted alicyclyl, substituted or non-substituted heterocyclyl, substituted or non-substituted heteroarylalkyl, substituted or non-substituted aryl, substituted or non-substituted aralkyl and R'₅-C(O)-; and
R"₂ is selected from the group consisting of -NR'₃R'₄, -OR'₃ and -SR'₃; where R'₃ and R'₄ are independently selected from the group consisting of H, substituted or non-substituted non-cyclic aliphatic group, substituted or non-substituted alicyclyl, substituted or non-substituted heterocyclyl, substituted or non-substituted heteroarylalkyl, substituted or non-substituted aryl and substituted or non-substituted aralkyl;
wherein R'₅ is selected from the group consisting of H, substituted or non-substituted non-cyclic aliphatic group, substituted or non-substituted alicyclyl, substituted or non-substituted aryl, substituted or non-substituted aralkyl, substituted or non-substituted heterocyclyl and substituted or non-substituted heteroarylalkyl.

The R"₁ and R"₂ groups are bound at the amino-terminal (*N*-terminal) and carboxy-terminal (*C*-terminal) ends of the peptide sequences, respectively.

In another particular embodiment, R"₁ is selected from the group consisting of H, and R'₅-CO-, wherein R'₅ is selected from the group consisting of substituted or unsubstituted C₁-C₂₄ alkyl radical, substituted or unsubstituted C₂-C₂₄ alkenyl radical, substituted or unsubstituted C₂-C₂₄ alkynyl radical, substituted or unsubstituted C₃-C₂₄ cycloalkyl radical, substituted or unsubstituted C₅-C₂₄ cycloalkenyl radical, substituted or unsubstituted C₈-C₂₄ cycloalkynyl radical, substituted or unsubstituted C₆-C₃₀ aryl radical, substituted or unsubstituted C₇-C₂₄ aralkyl radical, a substituted or unsubstituted heterocyclyl radical having 3-10 ring members, a substituted or unsubstituted heteroarylalkyl radical having 2 to 24 carbon atoms and having 1 to 3 atoms other than carbon wherein the alkyl chain is of 1 to 6 carbon atoms. Preferably, R"₁ is selected from the group consisting of H, acetyl, *tert*-butanoyl, hexanoyl, 2-methylhexanoyl, cyclohexanecarboxyl, octanoyl, decanoyl, lauroyl, myristoyl, palmitoyl, stearoyl, behenyl, oleoyl and linoleoyl. More preferably, R"₁ is H, acetyl, hexanoyl, octanoyl, lauroyl, myristoyl or palmitoyl.

In another particular embodiment, R"₂ is -NR'₃R'₄, -OR'₃ or -SR'₃ where R'₃ and R'₄ are independently selected from the group consisting of H, substituted or unsubstituted C₁-C₂₄ alkyl, substituted or unsubstituted C₂-C₂₄ alkenyl, substituted or unsubstituted C₂-C₂₄ alkynyl, substituted or unsubstituted C₃-C₂₄ cycloalkyl, substituted or unsubstituted C₅-C₂₄ cycloalkenyl, substituted or unsubstituted C₈-C₂₄ cycloalkynyl, substituted or unsubstituted C₆-C₃₀ aryl, substituted or unsubstituted C₇-C₂₄ aralkyl, a substituted or unsubstituted heterocyclyl having 3-10 ring members, and a substituted or unsubstituted heteroarylalkyl group having 2 to 24 carbon atoms and having 1 to 3 atoms other than carbon wherein the alkyl chain is of 1 to 6 carbon atoms. Optionally, R'₃ and R'₄ can be bound by means of a saturated or unsaturated carbon-carbon bond, forming a cycle with the nitrogen atom. Preferably, R"₂ is -NR'₃R'₄ or -OR'₃, wherein R'₃ and R'₄ are independently selected from the group consisting of H, substituted or unsubstituted C₁-C₂₄ alkyl, substituted or unsubstituted C₂-C₂₄ alkenyl, substituted or unsubstituted C₂-C₂₄ alkynyl, substituted or unsubstituted C₃-C₁₀ cycloalkyl, substituted or unsubstituted C₆-C₁₅ aryl and a substituted or unsubstituted heterocyclyl of 3-10 members, a substituted or unsubstituted heteroarylalkyl group with a ring having 3 to 10 members and an alkyl chain of 1 to 6 carbon atoms. More preferably, R'₃ and R'₄ are selected from the group consisting of H, methyl, ethyl, hexyl, dodecyl and hexadecyl. Even more preferably, R'₃ is H and R'₄ is selected from the group consisting of H, methyl, ethyl, hexyl, dodecyl and hexadecyl. According to an even more preferred embodiment, R"₂ is selected from -OH and -NH₂.

In another particular embodiment, R"₁ is selected from the group consisting of H, acetyl, lauroyl, myristoyl and palmitoyl, AA₁ is -L-Glu-, AA₂ is -L-Asp-, AA₃ is -L-Tyr-, AA₄ is -L-Tyr-, AA₅ is -L-Arg-, AA₆ is -L-Leu-, and R₂ is -NR'₃R'₄ or -OR'₃ wherein R'₃ and R'₄ are independently selected from the group consisting of H, methyl, ethyl, hexyl, dodecyl and hexadecyl, preferably R"₂ is -OH or -NH₂. More preferably, R"₁ is acetyl or palmitoyl and R"₂ is -NH₂. Even more preferably, p, r, s and t are 0.

In another particular embodiment, R"₁ is selected from the group consisting of H, acetyl, lauroyl, myristoyl and palmitoyl, AA₁ is -L-Pro-, AA₂ is -L-Asp-, AA₃ is -L-Tyr-, AA₄ is -L-Tyr-, AA₅ is -L-Lys-, AA₆ is -L-Leu-, and R₂ is -NR'₃R'₄ or -OR'₃ wherein R'₃ and R'₄ are independently selected from the group consisting of H, methyl, ethyl, hexyl, dodecyl and hexadecyl, preferably R"₂ is -OH or -NH₂. More preferably, R"₁ is acetyl or palmitoyl and R"₂ is -NH₂. Even more preferably, p, r, s and t are 0.

In another particular embodiment, R"₁ is selected from the group consisting of H, acetyl, lauroyl, myristoyl and palmitoyl, AA₁ is -L-Glu-, AA₂ is -L-Asp-, AA₃ is -L-Arg-, AA₄ is -L-Phe-, AA₅ is -L-Arg-, AA₆ is -L-Met-, and R₂ is -NR'₃R'₄ or -OR'₃ wherein R'₃ and R'₄ are independently selected from the group consisting of H, methyl, ethyl, hexyl, dodecyl and hexadecyl, preferably R"₂ is -OH or -NH₂. More preferably, R"₁ is acetyl or palmitoyl and R"₂ is -NH₂. Even more preferably, p, r, s and t are 0.

In another particular embodiment, R"₁ is selected from the group consisting of H, acetyl, lauroyl, myristoyl and palmitoyl, AA₁ is -L-Glu-, AA₂ is -L-Asp-, AA₃ is -L-Tyr-, AA₄ is -L-Tyr-, AA₅ is -L-Arg-, AA₆ is -L-Met-, and R₂ is -NR'₃R'₄ or -OR'₃ wherein R'₃ and R'₄ are independently selected from the group consisting of H, methyl, ethyl, hexyl, dodecyl or hexadecyl, preferably R"₂ is -OH or -NH₂. More preferably, R"₁ is acetyl or palmitoyl and R"₂ is -NH₂. Even more preferably, p, r, s and t are 0.

In another particular embodiment, R"₁ is selected from the group consisting of H, acetyl, lauroyl, myristoyl and palmitoyl, AA₁ is -L-Pro-, AA₂ is -L-Asp-, AA₃ is -L-Tyr-, AA₄ is -L-Tyr-, AA₅ is -L-Arg-, AA₆ is -L-Met-, and R₂ is -NR'₃R'₄ or -OR'₃ wherein R'₃ and R'₄ are independently selected from the group consisting of H, methyl, ethyl, hexyl, dodecyl and hexadecyl, preferably R"₂ is -OH or -NH₂. More preferably, R"₁ is acetyl or palmitoyl and R"₂ is -NH₂. Even more preferably, p, r, s and t are 0.

In another particular embodiment, R"₁ is selected from the group consisting of H, acetyl, lauroyl, myristoyl and palmitoyl, preferably R"₁ is selected from the group consisting of H, acetyl and palmitoyl, and R"₂ is selected from the group consisting of -OH or-NH₂.

In another particular embodiment, the peptide of general formula (IV) is selected from the group consisting of Ac-L-Glu-L-Asp-L-Tyr-L-Tyr-L-Arg-L-Leu-NH₂, Palm-L-Glu-L-Asp-L-Tyr-L-Tyr-L-Arg-L-Leu-NH₂, Ac-L-Pro-L-Asp-L-Tyr-L-Tyr-L-Lys-L-Leu-NH₂, Palm-L-Pro-L-Asp-L-Tyr-L-Tyr-L-Lys-L-Leu-NH₂, Ac-L-Glu-L-Asp-L-Arg-L-Phe-L-Arg-L-Met-NH₂, Palm-L-Glu-L-Asp-L-Arg-L-Phe-L-Arg-L-Met-NH₂, Ac-L-Glu-L-Asp-L-Tyr-L-Tyr-L-Arg-L-Met-NH₂, Palm-L-Glu-L-Asp-L-Tyr-L-Tyr-L-Arg-L-Met-NH₂, Ac-L-Pro-L-Asp-L-Tyr-L-Tyr-L-Arg-L-Met-NH₂ and Palm-L-Pro-L-Asp-L-Tyr-L-Tyr-L-Arg-L-Met-NH₂.

Within the scope of this invention are also included the cosmetically acceptable salts of the peptides of the cosmetic composition of the method of this invention. The term "cosmetically acceptable salts" in this invention means a salt generally recognized for use in animals and more particularly in humans, including the salts used to form base addition salts, either inorganic, such as, for example, and without limitation thereto, lithium, sodium, potassium, calcium, magnesium, manganese, copper, zinc or aluminum, among others, or organic such as, for example and not limited thereto, ethylamine, diethylamine, ethylenediamine, ethanolamine, diethanolamine, arginine, lysine, histidine or piperazine, among others, or acid addition salts, either organic such as for example and without limitation thereto, acetate, citrate, lactate, malonate, maleate, tartrate, fumarate, benzoate, aspartate, glutamate, succinate, oleate, trifluoroacetate, oxalate, pamoate or gluconate, among others, or inorganic, such as, for example, and not limited thereto, chloride, sulfate, borate or carbonate, among others. The nature of the salt is not critical, provided that it is cosmetically acceptable. The cosmetically acceptable salts of the peptides of the cosmetic composition of the method of this invention can be obtained by conventional methods, well known in the state of the art *[*Berge, S.M., Bighley, L.D., and Monkhouse, D.C. (1977) "Pharmaceutical Salts" J. Pharm. Sci 66:1-19*].*

Additionally, the peptides of the cosmetic composition of the method of this invention can undergo reversible chemical modifications to enhance their bioavailability and ease of crossing of the blood-brain barrier or the epithelial tissue.

The peptides comprised in the cosmetic composition of the method of this invention can be administered by any means that produces contact of the peptides with their action site in the body of a mammal, preferably human beings. The cosmetic composition can be prepared by conventional methods known by a person skilled in the art *["*Harry's Cosmeticology", Eight [sic] edition (2000) Rieger M.M., ed., New York Chemical Pub., NY, US*; "*Remington: The Science and Practice of Pharmacy", Twentieth edition (2003) Genaro A.R., ed., Lippincott Williams & Wilkins, Philadelphia, US*].*

The peptides comprised in the cosmetic composition of the method of this invention have variable solubility in water, depending on the nature of their sequences or the possible modifications in their amino- and/or carboxy-terminal that they have. Therefore, the peptides can be incorporated into the cosmetic composition by means of an aqueous solution, and those that are not soluble in water can be solubilized in cosmetically acceptable conventional solvents such as, for example, and not limited thereto, ethanol, propanol, isopropanol, propylene glycol, glycerin, butylene glycol or polyethylene glycol or any combination thereof.

The effective amount of peptides comprised in the cosmetic composition of the method of this invention, their stereoisomers, mixtures thereof or their cosmetically acceptable salts, as well as their dosage, will depend on a number of factors including age, subject condition, route, frequency of administration and the particular nature of the peptides used.

"Effective amount" means a non-toxic but sufficient amount of at least one peptide to provide the desired effect. Any peptide of general formula (I) and/or (II) of the cosmetic composition of the method of this invention is used at concentrations effective to achieve the desired effect; preferably, in reference to the total weight of the composition, between 0.00000001% (in weight) and 20% (in weight); preferably between 0.000001% (in weight) and 20% (in weight), more preferably between 0.0001% (in weight) and 10% (in weight) and even more preferably between 0.0001% (in weight) and 5% (in weight).

In another particular embodiment, the peptides of general formula (I) and/or (II) comprised in the cosmetic composition of the method of this invention can also be incorporated into delivery systems and/or sustained release systems.

The term "delivery systems" refers to a diluent, adjuvant, excipient or carrier with which the peptide derivative of the invention is administered. These carriers can be liquids such as water, oils and surfactants, including those of petroleum, animal, vegetable or synthetic origin, such as, for example, and not limited thereto, peanut oil, soybean oil, mineral oil, sesame oil, castor oils, polysorbates, sorbitan esters, ether sulfates, sulfates, betaines, glucosides, maltosides, fatty alcohols, nonoxynol, poloxamer, polyoxyethylenes, polyethylene glycols, dextrose, glycerol, digitonin and the like. *"Remington's Pharmaceutical Sciences"* by E.W. Martin describes diluents, adjuvants or excipients as appropriate carriers.

The term "sustained release" is used in the conventional sense, referring to a delivery system for a compound that provides gradual release of said compound for a time period and preferably, though not necessarily, with constant release levels of the compound over a period of time.

Examples of delivery or sustained release systems are liposomes, mixed liposomes, oleosomes, niosomes, miniparticles, milliparticles, microparticles, nanoparticles and solid lipid nanoparticles, nanostructured lipid carriers, sponges, cyclodextrins, vesicles, micelles, mixed micelles of surfactants, surfactant-phospholipid mixed micelles, millispheres, microspheres and nanospheres, lipospheres, millicapsules, microcapsules and nanocapsules, as well as microemulsions and nanoemulsions, which can be added to achieve a greater penetration of the active principle and/or improve its pharmacokinetic and pharmacodynamic properties.

The sustained release systems can be prepared by methods known in the prior art, and the compositions which contain them can be administered, for example, by topical administration, including adhesive patches, non-adhesive patches and microelectric patches, or by systemic administration, for example subcutaneous implantation or injection, or direct implantation or injection into a specific body part, and preferably should release a relatively constant quantity of the peptides of the invention. The amount of peptide contained in the sustained release system will depend, for example, on where the composition is to be administered, the kinetics and duration of the release of the peptide of the cosmetic composition of the method of this invention, as well as the frequency of administration and the particular nature of the peptides to be used.

In another particular embodiment, the peptides comprised in the cosmetic composition of the method of this invention can also be adsorbed on solid organic polymers, or solid mineral carriers such as, but not limited to, talc, bentonite, silica, starch or maltodextrin, among others.

In another particular embodiment, the cosmetic composition of the method of this invention can also be incorporated into fabrics, non-woven fabrics and medical devices that are in direct contact with the skin, such that they release the peptides either by biodegradation of the anchoring system to the fabric, non-woven fabric or medical device or by the friction of these ones with the body, body moisture, the pH of the skin or by body temperature. Likewise, fabrics and non-woven fabrics can be used to make garments that are in direct contact with the body.

Examples of fabrics, non-woven fabrics, garments, medical devices and means of inmobilizing the peptides to them, including the delivery systems and/or sustained release systems described above can be found described in literature and are known in the state of the art *[*Schaab C.K. (1986) "Impregnating Fabrics With Microcapsules", HAPPI May 1986*;* Nelson G. (2002) "Application of microencapsulation in textiles" Int. J. Pharm. 242:55-62*; "*Biofunctional Textiles and the Skin" (2006) Curr. Probl. Dermatol. v.33, Hipler U. C. and Elsner P., eds. S. Karger AG, Basel, Switzerl*and;* Malcom R.K., McCullagh S.D., Woolfson A.D., Gorman S.P., Jones D.S. and Cuddy J. (2004) "Controlled release of a model antibacterial drug from a novel self lubricating silicone biomaterial" J. Cont. Release 97:313-320*].* The preferred fabrics, non-woven fabrics, garments and medical devices are bandages, gauzes, wipes, adhesive patches, non-adhesive patches, microelectric patches and/or face masks.

In another particular embodiment, the cosmetic composition of the method of this invention can be used in different types of formulations for topical or transdermal application which will optionally contain the acceptable excipients necessary for the formulation of the desired dosage form *[*Faulí i Trillo C. (1993) in "Tratado de Farmacia Galenica", Luzán 5, S.A. Ediciones, Madrid*].*

The cosmetic composition can be produced in any solid, liquid or semisolid formulation, such as and not restricted to, creams, multiple emulsions such as and not restricted to, oil and/or silicone in water emulsions, water-in-oil and/or silicone emulsions, water/oil/water or water/silicone/water type emulsions, and oil/water/oil or silicone/water/silicone type emulsions, anhydrous compositions, aqueous dispersions, oils, milks, balsams, foams, lotions, gels, cream gels, hydroalcoholic solutions, hydroglycolic solutions, hydrogels, liniments, sera, soaps, shampoos, conditioners, serums, polysaccharide films, ointments, mousses, pomades, powders, bars, pencils and sprays or aerosols (sprays), including leave-on and rinse-off formulations. These formulations can be incorporated using techniques known by the person skilled in the art into different types of solid accessories such as and not restricted to, capsules, vials, syringes, pre-loaded syringes, wipes, adhesive patches, non-adhesive patches, microelectric patches or face masks, or they can be incorporated into different make-up products such as make-up foundation, such as fluid foundations and compact foundations, make-up removal lotions, make-up removal milks, under-eye concealers, eye shadows, lipsticks, lip protectors, lip gloss and powders among others.

In another particular embodiment, the cosmetic composition of the method of this invention can additionally include agents that enhance the percutaneous absorption for topical or transdermal application of the peptides of general formula (I) and/or (II), their stereoisomers, mixtures thereof and/or their cosmetically acceptable salts, such as, for example, but not limited thereto, dimethylsulfoxide, dimethylacetamide, dimethylformamide, surfactants, azone (1-dodecylazacycloheptan-2-one), alcohol, acetone, propylene glycol or polyethylene glycol, among others. Furthermore, the cosmetic composition of the method of this invention can be applied by topical or transdermal application to local areas to be treated by means of iontophoresis, sonophoresis, electroporation, microelectric patches, mechanical pressure, osmotic pressure gradient, occlusive cure, microinjections or needle-free injections by means of pressure, such as injections by oxygen pressure, or any combination thereof, to achieve a greater penetration of the peptides of the cosmetic composition of the method of this invention.

The composition of the method of this invention can also be administered, in addition to the topical or transdermal route, by any other appropriate means, e.g. by enteral or parenteral route, which will include the acceptable excipients necessary for formulation in the desired dosage form. A review of the different dosage forms of the active ingredients and excipients needed to obtain them can be found, for example, in the *"*Tratado de Farmacia Galenica", C. Fauli i Trillo, 1993, Luzán 5, S.A. Ediciones, Madrid*.*

In the context of this invention, the terms "enteral or parenteral" include oral, nasal, inhalational, rectal routes, adhesive or non-adhesive patches, subcutaneous, intradermal, intravascular injections, such as intravenous, intramuscular, intraarterial, intravitreal, spinal, intracranial, intraarticular, intrathecal and intraperitoneal, as well as any similar injection or infusion technique.

In another particular embodiment, the cosmetic composition of the method of this invention further comprises a cosmetically effective amount of at least one additional active agent commonly used in compositions for the treatment and/or care of the skin such as and not restricted to, cAMP (cyclic adenosine monophosphate) synthesis stimulating agents, elastase inhibiting agents, matrix metalloproteinases inhibiting agents, melanin synthesis stimulating or inhibiting agents, whitening or depigmenting agents, propigmenting agents, self-tanning agents, antiaging agents, NO-synthase inhibiting agents, 5α-reductase inhibiting agents, lysyl- and/or prolyl hydroxylase inhibiting agents, antioxidants, free radical scavengers and/or agents against atmospheric pollution, reactive carbonyl species scavengers, anti-glycation agents, antihistamine agents, antiviral agents, antiparasitic agents, emulsifiers, emollients, organic solvents, liquid propellants, skin and/or hair conditioners such as humectants, substances that retain moisture, alpha hydroxyacids, beta hydroxyacids, moisturizers, epidermal hydrolytic enzymes, vitamins, pigments or colorants, dyes, gelling polymers, thickeners, surfactants, softening agents, anti-wrinkle agents, agents able to reduce or treat bags under the eyes, exfoliating agents, antimicrobial agents, antifungal agents, fungistatic agents, bactericidal agents, bacteriostatic agents, agents stimulating the synthesis of dermal or epidermal macromolecules and/or capable of inhibiting or preventing their degradation, such as for example collagen synthesis-stimulating agents, elastin synthesis-stimulating agents, decorin synthesis-stimulating agents, laminin synthesis-stimulating agents, defensin synthesis-stimulating agents, chaperone synthesis-stimulating agents, aquaporin synthesis-stimulating agents, hyaluronic acid synthesis-stimulating agents, fibronectin synthesis-stimulating agents, sirtuin synthesis-stimulating agents, agents stimulating the synthesis of lipids and components of the stratum corneum (ceramides, fatty acids, etc.), agents that inhibit collagen degradation, other agents that inhibit elastin degradation, agents that inhibit serine proteases such as cathepsin G, agents stimulating fibroblast proliferation, agents stimulating keratinocyte proliferation, agents stimulating adipocyte proliferation, agents stimulating melanocyte proliferation, agents stimulating keratinocyte differentiation, agents stimulating adipocyte differentiation, agents that inhibit acetylcholinesterase, skin relaxant agents, agents that inhibit acetylcholine receptors aggregation, agents that inhibit muscle contraction, glycosaminoglycan synthesis-stimulating agents, antihyperkeratosis agents, comedolytic agents, antipsoriasis agents, DNA repair agents, DNA protecting agents, stabilizers, anti-itching agents, agents for the treatment and/or care of sensitive skin, firming agents, anti-stretch mark agents, binding agents, agents regulating sebum production, lipolytic agents or agents stimulating lipolysis, anti-cellulite agents, antiperspirant agents, agents stimulating healing, coadjuvant healing agents, agents stimulating reepithelialization, coadjuvant reepithelialization agents, cytokine growth factors, calming agents, anti-inflammatory agents, anesthetic agents, agents acting on capillary circulation and/or microcirculation, agents stimulating angiogenesis, agents that inhibit vascular permeability, venotonic agents, agents acting on cell metabolism, agents to improve dermal-epidermal junction, agents inducing hair growth, hair growth inhibiting or retardant agents, preservatives, perfumes, chelating agents, vegetable extracts, essential oils, marine extracts, agents obtained from a biofermentation process, mineral salts, cell extracts and sunscreens (organic or mineral photoprotective agents active against ultraviolet A and/or B rays) among others, provided they are physically and chemically compatible with the other components of the composition and especially with the peptides of general formulas (I) and (II) contained in the composition of this invention. Furthermore, the nature of these additional ingredients should not unacceptably alter the benefits of the peptides of the composition of the method of this invention. The nature of these additional ingredients can be synthetic or natural, such as vegetable extracts, or obtained by a biofermentation process. Additional examples can be found in the CTFA International Cosmetic Ingredient Dictionary & Handbook, 12th Edition (2008*).*

In a preferred embodiment, the cosmetic composition of the method of this invention additionally comprises a cosmetically effective amount of at least one extract which is an anti-wrinkle agent and/or an antiaging agent such as and not restricted to the extracts of *Vitis vinifera, Rosa canina, Curcuma longa, Iris pallida, Theobroma cacao, Ginkgo biloba, Leontopodium Alpinum* or *Dunaliella salina* among others or, in addition, at least one synthetic compound or bio-fermentation product which is an anti-wrinkle agent and/or an antiaging agent such as and not restricted to Matrixyl® [INCI: Palmitoyl Pentapeptide-4], Matrixyl 3000® [INCI: Palmitoyl Tetrapeptide-7, Palmitoyl Oligopeptide], Essenskin™ [INCI: calcium hydroxymethionine], Renovage [INCI: teprenone] or Dermaxyl® [INCI: Palmitoyl Oligopeptide] marketed by Sederma/Croda, Vialox® [INCI: Pentapeptide-3], Syn®-Ake [INCI: Dipeptide Diaminobutyroyl Benzylamide Diacetate], Syn®-Coll [INCI: Palmitoyl Tripeptide-5], Phytaluronate [INCI: Locust Bean (Ceratonia Siliqua) Gum] or Preregen® [INCI: Glycine Soya (Soybean) Protein, Oxido Reductases] marketed by Pentapharm/DSM, Myoxinol™ [INCI: Hydrolyzed Hibiscus Esculentus Extract], Syniorage™ [INCI: Acetyl Tetrapeptide-11], Dermican™ [INCI: Acetyl Tetrapeptide-9] or DN-AGE™ LS [INCI: Cassia Alata leaf Extract] marketed by Laboratoires Serobiologiques/Cognis, Algisum C® [INCI: Methylsilanol Mannuronate] or Hydroxyprolisilane CN® [INCI: Methylsilanol Hydroxyproline Aspartate] marketed by Exsymol, Aldenine® [INCI: Hydrolized wheat protein, hydrolized soy protein, Tripeptide-1], Preventhelia™ [INCI: Diaminopropionoyl Tripeptide-33], Decorinyl™ [INCI: Tripeptide-10 Citrulline], Trylagen™ [INCI: Pseudoalteromonas Ferment Extract, Hydrolyzed Wheat Protein, Hydrolyzed Soy Protein, Tripeptide-10 Citrulline, Tripeptide-1], Eyeseryl® [INCI: Acetyl Tetrapeptide-5], Peptide AC29 [INCI: Acetyl Tripeptide-30 Citrulline], Lipochroman-6 [INCI: Dimethylmethoxy Chromanol], Chromabright™ [INCI: Dimethylmethoxy Chromanyl Palmitate], Antarcticine® [INCI: Pseudoalteromonas Ferment Extract], Vilastene™ [INCI: Lysine HCl, Lecithin, Tripeptide-10 Citrulline], Inyline™ [INCI: Acetyl Hexapeptide-30], Relistase™ [INCI: Acetylarginyltriptophyl Diphenylglycine], Thermostressine™ [INCI: Acetyl Tetrapeptide-22] marketed by Lipotec, Kollaren® [INCI: Tripeptide-1, Dextran] marketed by Institut Europeen de Biologie Cellulaire/Unipex Group, Collaxyl® IS [INCI: Hexapeptide-9], Laminixyl IS™ [INCI: Heptapeptide], Orsirtine™ GL [INCI: Oryza Sativa (Rice) Extract], D'Orientine™ IS [INCI: Phoenix Dactylifera (Date) Seed Extract], Phytoquintescine™ [INCI: Einkorn (Triticum Monococcum) Extract] or Quintescine™ IS [INCI: Dipeptide-4] marketed by Vincience/ISP, BONT-L-Peptide [INCI: Palmitoyl Hexapeptide-19] marketed by Infinitec Activos, Deepaline™ PVB [INCI: Palmitoyl hydrolyzed Wheat Protein] or Sepilift® DPHP [INCI: Dipalmitoyl Hydroxyproline] marketed by Seppic, Gatuline® Expression [INCI: Acmella oleracea Extract], Gatuline® In-Tense [INCI: Spilanthes Acmella Flower Extract] or Gatuline® Age Defense 2 [INCI: Juglans Regia (Walnut) Seed Extract] marketed by Gattefossé, Thalassine™ [INCI: Algae Extract] marketed by Biotechmarine, ChroNOline™ [INCI: Caprooyl Tetrapeptide-3] or Thymulen-4 [INCI: Acetyl Tetrapeptide-2] marketed by Atrium/Unipex Innovations, EquiStat [INCI: Pyrus Malus Fruit Extract, Glycine Soja Seed Extract] or Juvenesce [INCI: Ethoxydiglycol and Caprylic Triglycerid, Retinol, Ursolic Acid, Phytonadione, Ilomastat] marketed by Coletica, Ameliox [INCI: Carnosine, Tocopherol, Silybum Marianum Fruit Extract] or PhytoCellTec Malus Domestica [INCI: Malus Domestica Fruit Cell Culture] marketed by Mibelle Biochemistry, Bioxilift [INCI: Pimpinella Anisum Extract] or SMS Anti-Wrinkle® [INCI: Annona Squamosa Seed Extract] marketed by Silab, calcium channel antagonists such as and not restricted to, alverine, manganese or magnesium salts, magnesium gluconate, certain secondary or tertiary amines, retinol and its derivatives, idebenone and its derivatives, Coenzyme Q10 and its derivatives, boswellic acid and its derivatives, GHK and its derivatives, carnosine and its derivatives, DNA repair enzymes such as and not restricted to, photolyase, T4 endonuclease V, or chloride channel agonists among others.

In another particular embodiment, the cosmetic composition of the method of this invention additionally includes acceptable carriers and/or auxiliary agents necessary for the administration of the composition in the desired manner. Among the carriers and/or auxiliary agents are included excipients, thickeners, diluents, solvents, dispersants or adjuvants known to the expert of the art. Thickeners include, but are not limited to, water-soluble polymers such as those selected from the group consisting of modified celluloses, methylcellulose, ethylcellulose, hydroxyethylcellulose, hydroxyethylmethylcellulose, hydroxypropylcellulose, hydroxypropylmethylcellulose and carboxymethylcellulose, dextrans, gelatins, collagen, polyethylene glycol or polyvinyl pyrrolidone. Diluents and solvents include, but are not limited to, those selected from the group consisting of ethanol, polyethylene glycol, glycofurol, *N*-methyl-2-pyrrolidone, glycerol, propanediol, polypropylene glycol, benzyl alcohol or dimethylsulfoxide. Dispersants include, but are not limited to, surfactants selected from the group consisting of monoesters of fatty acids of polyoxyethylene sorbitan (Tween®, Emalex, Nikkol®, Hodag, Dacol or Liposorb®), fatty acid monoesters of sorbitan (Span®), 15-hydroxystearate polyethylene glycol (Solutol® HS15), fatty acid esters of polyethylene glycol (Crodet, Cithrol, Kessco®, Nikkol®, Mapeg®, Myrj, Tagat®, Aldo®, Capmul®, Glycerox, Lactomul®, or Emerest®), esters of glycol polyoxyethylene (Emulphor®), polyethoxylated castor oils (Cremophor®, Emalex, Eumulgin®, Nikkol® or Simusol®), fatty acid esters of polyglycerol (Nikkol Decaglyn, Polymuls, Caprol®), polyethylene glycol ethers (Volpo or Brij®), poloxamer (Lutrol® or Pluronic®), phenyl ethers of polyoxyethylene (Triton® or Igepal®), or mixtures thereof. Preferably, the cosmetic composition of the method of this invention also contains one or more acceptable excipients such as humectants, pH buffers, preservatives, bactericidal and fungicidal agents, absorption retardants, absorption accelerators, or any other excipient known to the expert of the art.

The administration of the cosmetic composition of the method of this invention can be started before, as a pre-treatment, simultaneously, as a co-treatment, and/or just after, as a post-treatment, performing Botulinum toxin injections. The frequency of the administration of the cosmetic composition of the method of this invention can vary widely, depending on the needs of each subject, with recommendation for a range of administration from once a week to ten times a day, preferably from twice a week to four times a day, more preferably from three times a week to three times a day, even more preferably once or twice a day. In a most preferred embodiment, the method of this invention comprises the administration of Botulinum toxin, followed by the administration of the cosmetic composition of the method of this invention twice a day until the next Botulinum toxin administration.

In a particular embodiment, the wrinkle reduction average obtained by the method of this invention after 2 months of treatment is higher than 50% in relation to the maximum of the wrinkle reduction average, particularly higher than 60%. The wrinkle reduction average after 4 months of treatment obtained by the method of this invention is higher than 25% in relation to the maximum of the wrinkle reduction average, particularly higher than 35%. The wrinkle reduction average after 6 months of treatment obtained by the method of this invention is higher than 10% in relation to the maximum of the wrinkle reduction average, particularly higher than 20%.

In a second aspect, this invention refers to a kit for skin antiaging treatment, comprising:
a. Botulinum toxin,
b. and at least one cosmetic composition comprising a cosmetically effective amount of at least one peptide which contains a sequence of 6 to 40 adjacent amino acids contained in the amino acid sequence of the SNAP-25 protein, defined by SEQ ID No.1, and according to the general formula (I):

   R₁-AA-R₂ (I)

   its stereoisomers, mixtures thereof, and/or its cosmetically acceptable salts thereof, in which AA is a sequence of 6 to 40 adjacent amino acids contained in the amino acid sequence SEQ ID No.1;
   and/or at least one enkephalin-derived peptide of general formula (II): 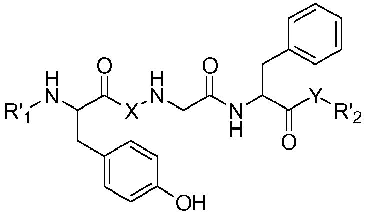 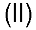 its stereoisomers, mixtures thereof, and/or its cosmetically acceptable salts thereof,
wherein:
X and Y are independently selected from the group consisting of natural amino acids and non-natural amino acids;
R₁ and R'₁ are independently selected from the group consisting of H, substituted or non-substituted non-cyclic aliphatic group, substituted or non-substituted alicyclyl, substituted or non-substituted heterocyclyl, substituted or non-substituted heteroarylalkyl, substituted or non-substituted aryl, substituted or non-substituted aralkyl and R₅-C(O)-; and
R₂ and R'₂ are independently selected from the group consisting of -NR₃R₄, -OR₃ and -SR₃; where R₃ and R₄ are independently selected from the group consisting of H, substituted or non-substituted non-cyclic aliphatic group, substituted or non-substituted alicyclyl, substituted or non-substituted heterocyclyl, substituted or non-substituted heteroarylalkyl, substituted or non-substituted aryl and substituted or non-substituted aralkyl;
wherein R₅ is selected from the group consisting of H, substituted or non-substituted non-cyclic aliphatic group, substituted or non-substituted alicyclyl, substituted or non-substituted aryl, substituted or non-substituted aralkyl, substituted or non-substituted heterocyclyl and substituted or non-substituted heteroarylalkyl;
and at least one cosmetically acceptable excipient or adjuvant.

The R₁, R'₁ and R₂, R'₂ groups are bound at the amino-terminal (*N*-terminal) and carboxy-terminal (*C*-terminal) ends of the peptide sequences, respectively.

In one particular embodiment, the skin antiaging treatment is a treatment for reducing or eliminating wrinkles of the facial and/or neck skin, preferably expression wrinkles.

In another particular embodiment, the preferred structures of the peptides represented in the general formula (II) are those where X is -Gly-, -Ala- or -Ser-, and more preferably -D-Ala- or -D-Ser-.

In another particular embodiment, the preferred structures of the peptides represented in the general formula (II) are those where Y is -Leu- or -Met-, and more preferably-L-Leu- or -L-Met-.

In another particular embodiment, R₁ and R'₁ are independently selected from the group consisting of H, a polymer of general formula (III) 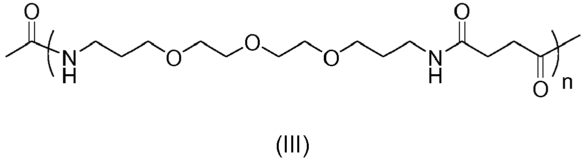 wherein n ranges between 1 to 100, preferably between 1 and 5, and R₅-CO-, wherein R₅ is selected from the group consisting of substituted or unsubstituted C₁-C₂₄ alkyl radical, substituted or unsubstituted C₂-C₂₄ alkenyl radical, substituted or unsubstituted C₂-C₂₄ alkynyl radical, substituted or unsubstituted C₃-C₂₄ cycloalkyl radical, substituted or unsubstituted C₅-C₂₄ cycloalkenyl radical, substituted or unsubstituted C₈-C₂₄ cycloalkynyl radical, substituted or unsubstituted C₆-C₃₀ aryl radical, substituted or unsubstituted C₇-C₂₄ aralkyl radical, a substituted or unsubstituted heterocyclyl radical having 3-10 ring members, a substituted or unsubstituted heteroarylalkyl radical having 2 to 24 carbon atoms and having 1 to 3 atoms other than carbon wherein the alkyl chain is of 1 to 6 carbon atoms. Preferably, R₁ and R'₁ are independently selected from the group consisting of H, acetyl, *tert-*butanoyl, hexanoyl, 2-methylhexanoyl, cyclohexanecarboxyl, octanoyl, decanoyl, lauroyl, myristoyl, palmitoyl, stearoyl, behenyl, oleoyl and linoleoyl. More preferably, R₁ and R'₁ are independently selected from the group consisting of H, acetyl, hexanoyl, octanoyl, lauroyl, myristoyl or palmitoyl.

In another particular embodiment, R₂ and R'₂ are independently selected from the group consisting of -NR₃R₄, -OR₃ or -SR₃ where R₃ and R₄ are independently selected from the group consisting of H, substituted or unsubstituted C₁-C₂₄ alkyl, substituted or unsubstituted C₂-C₂₄ alkenyl, substituted or unsubstituted C₂-C₂₄ alkynyl, substituted or unsubstituted C₃-C₂₄ cycloalkyl, substituted or unsubstituted C₅-C₂₄ cycloalkenyl, substituted or unsubstituted C₈-C₂₄ cycloalkynyl, substituted or unsubstituted C₆-C₃₀ aryl, substituted or unsubstituted C₇-C₂₄ aralkyl, a substituted or unsubstituted heterocyclyl having 3-10 ring members, and a substituted or unsubstituted heteroarylalkyl group having 2 to 24 carbon atoms and having 1 to 3 atoms other than carbon wherein the alkyl chain is of 1 to 6 carbon atoms and a polymer of general formula (III) wherein n ranges between 1 and 100, preferably between 1 and 5.

Optionally, R₃ and R₄ can be bound by means of a saturated or unsaturated carbon-carbon bond, forming a cycle with the nitrogen atom. Preferably, R₂ and R'₂ are independently selected from the group consisting of -NR₃R₄ or -OR₃, wherein R₃ and R₄ are independently selected from the group consisting of H, substituted or unsubstituted C₁-C₂₄ alkyl, substituted or unsubstituted C₂-C₂₄ alkenyl, substituted or unsubstituted C₂-C₂₄ alkynyl, substituted or unsubstituted C₃-C₁₀ cycloalkyl, substituted or unsubstituted C₆-C₁₅ aryl and a substituted or unsubstituted heterocyclyl of 3-10 members, a substituted or unsubstituted heteroarylalkyl group with a ring having 3 to 10 members and an alkyl chain of 1 to 6 carbon atoms and a polymer of general formula (III) wherein n ranges between between 1 and 100, preferably between 1 and 5. More preferably, R₃ and R₄ are selected from the group consisting of H, methyl, ethyl, hexyl, dodecyl and hexadecyl. Even more preferably, R₃ is H and R₄ is selected from the group consisting of H, methyl, ethyl, hexyl, dodecyl and hexadecyl. According to an even more preferred embodiment, R₂ and R'₂ are independently selected from the group consisting of -OH and -NH₂.

In the context of this invention "amino acid sequence derived from the amino acid sequence of the SNAP-25 protein" means any amino acid sequence or fragments of the amino acid sequence of the SNAP-25 protein, defined by the SEQ ID No.1, or any amino acid sequence that differs from the sequence SEQ ID No.1 by mutation, insertion, deletion or substitution of at least one amino acid, or by degeneration of the genetic code, provided that it corresponds to a peptide that possesses the activity of the SNAP-25 protein.

Among the peptides derived from the amino acid sequence of SNAP-25 defined by SEQ ID No.1 included in the cosmetic composition of the method of this invention, preferred sequences are those that possess a sequence of adjacent amino acids contained in the sequence of the amino-terminal region of the SNAP-25 protein defined by SEQ ID No.2 or the carboxy-terminal region of the SNAP-25 protein defined by SEQ ID No.3, more preferably in the region between residues 10 to 22, defined by SEQ ID No.4, or contained in the region between residues 25 to 40, defined by SEQ ID No.5, or contained in the region between residues 65 to 81, defined by SEQ ID No.6, or contained in the region between residues 181 to 206, defined by SEQ ID No.7, more precisely contained in the region between residues 12 to 19, defined by SEQ ID No.8, or contained in the region between residues 26 to 38, defined by SEQ ID No.9 or contained in the region between residues 68 to 79, defined by SEQ ID No.10 or specifically contained in the region between residues 12 to 17, defined by SEQ ID No.11, and a sequence of 7 to 12 adjacent amino acids contained in SEQ ID No.4, wherein said peptide comprises the amino acid sequence of SEQ ID No.11.

In particular, preferred amino acid sequences are preferably those sequences selected from the group consisting of SEQ ID No.4, SEQ ID No.5, SEQ ID No.6, SEQ ID No.7, SEQ ID No.8, SEQ ID No.9, SEQ ID No.10, SEQ ID No.11, SEQ ID No.12, SEQ ID No.13, SEQ ID No.14, SEQ ID No.15, SEQ ID No.16, SEQ ID No.17, SEQ ID No.18, SEQ ID No.19, SEQ ID No.20, SEQ ID No.21, SEQ ID No.22, SEQ ID No.23, SEQ ID No.24, SEQ ID No.25, SEQ ID No.26, SEQ ID No.27, SEQ ID No.28, SEQ ID No.29, SEQ ID No.30, SEQ ID No.31 and SEQ ID No.32. More preferably, amino acid sequences are those sequences selected from the group consisting of SEQ ID No.4, SEQ ID No.5, SEQ ID No.6, SEQ ID No.7, SEQ ID No.8, SEQ ID No.11 and SEQ ID No.26.

Among the peptides defined by the general formula (II) included in the cosmetic composition of the kit of the method of this invention, preferred amino acid sequences are preferably those sequences selected from the group consisting of SEQ ID No.33, SEQ ID No.34, SEQ ID No.35, SEQ ID No.36, SEQ ID No.37 and SEQ ID No.38. More preferably, amino acid sequences are those sequences selected from the group consisting of SEQ ID No.33 and SEQ ID No.35.

The cosmetic composition of the kit of the method of the invention can further comprise a cosmetically effective amount of at least one peptide of general formula (IV):

R"₁-Aₚ-Bᵣ-AA₁-AA₂-AA₃-AA₄-AA₅-AA₆-Cₛ-Dₜ-R"₂ (IV)

its stereoisomers, mixtures thereof, and/or its cosmetically acceptable salts thereof, wherein:
AA₁ is selected from the group consisting of -Asp-, -Glu- and -Pro-;
AA₂ is -hasp-;
AA₃ is selected from the group consisting of -Tyr- and -Arg-;
AA₄ is selected from the group consisting of -Phe- and -Tyr-;
AA₅ is selected from the group consisting of -Arg- and -Lys-;
AA₆ is selected from the group consisting of -Leu- and -Met-; A, B, C and D are independently selected from the group consisting of natural amino acids and non-natural amino acids;
p, r, s and t are independently selected and range between 0 and 1;
R"₁ is selected from the group consisting of H, substituted or non-substituted non-cyclic aliphatic group, substituted or non-substituted alicyclyl, substituted or non-substituted heterocyclyl, substituted or non-substituted heteroarylalkyl, substituted or non-substituted aryl, substituted or non-substituted aralkyl and R'₅-C(O)-; and
R"₂ is selected from the group consisting of -NR'₃R'₄, -OR'₃ and -SR'₃; where R'₃ and R'₄ are independently selected from the group consisting of H, substituted or non-substituted non-cyclic aliphatic group, substituted or non-substituted alicyclyl, substituted or non-substituted heterocyclyl, substituted or non-substituted heteroarylalkyl, substituted or non-substituted aryl and substituted or non-substituted aralkyl;
wherein R'₅ is selected from the group consisting of H, substituted or non-substituted non-cyclic aliphatic group, substituted or non-substituted alicyclyl, substituted or non-substituted aryl, substituted or non-substituted aralkyl, substituted or non-substituted heterocyclyl and substituted or non-substituted heteroarylalkyl.

The R"₁ and R"₂ groups are bound at the amino-terminal (*N*-terminal) and carboxy-terminal (*C*-terminal) ends of the peptide sequences, respectively.

In another particular embodiment, R"₁ is selected from the group consisting of H, preferably between 1 and 5, and R'₅-CO-, wherein R'₅ is selected from the group consisting of substituted or unsubstituted C₁-C₂₄ alkyl radical, substituted or unsubstituted C₂-C₂₄ alkenyl radical, substituted or unsubstituted C₂-C₂₄ alkynyl radical, substituted or unsubstituted C₃-C₂₄ cycloalkyl radical, substituted or unsubstituted C₅-C₂₄ cycloalkenyl radical, substituted or unsubstituted C₈-C₂₄ cycloalkynyl radical, substituted or unsubstituted C₆-C₃₀ aryl radical, substituted or unsubstituted C₇-C₂₄ aralkyl radical, a substituted or unsubstituted heterocyclyl radical having 3-10 ring members, a substituted or unsubstituted heteroarylalkyl radical having 2 to 24 carbon atoms and having 1 to 3 atoms other than carbon wherein the alkyl chain is of 1 to 6 carbon atoms. Preferably, R"₁ is selected from the group consisting of H, acetyl, *tert*-butanoyl, hexanoyl, 2-methylhexanoyl, cyclohexanecarboxyl, octanoyl, decanoyl, lauroyl, myristoyl, palmitoyl, stearoyl, behenyl, oleoyl and linoleoyl. More preferably, R"₁ is H, acetyl, hexanoyl, octanoyl, lauroyl, myristoyl or palmitoyl.

In another particular embodiment, R"₂ is -NR'₃R'₄, -OR'₃ or -SR'₃ where R'₃ and R'₄ are independently selected from the group consisting of H, substituted or unsubstituted C₁-C₂₄ alkyl, substituted or unsubstituted C₂-C₂₄ alkenyl, substituted or unsubstituted C₂-C₂₄ alkynyl, substituted or unsubstituted C₃-C₂₄ cycloalkyl, substituted or unsubstituted C₅-C₂₄ cycloalkenyl, substituted or unsubstituted C₈-C₂₄ cycloalkynyl, substituted or unsubstituted C₆-C₃₀ aryl, substituted or unsubstituted C₇-C₂₄ aralkyl, a substituted or unsubstituted heterocyclyl having 3-10 ring members, and a substituted or unsubstituted heteroarylalkyl group having 2 to 24 carbon atoms and having 1 to 3 atoms other than carbon wherein the alkyl chain is of 1 to 6 carbon atoms. Optionally, R'₃ and R'₄ can be bound by means of a saturated or unsaturated carbon-carbon bond, forming a cycle with the nitrogen atom. Preferably, R"₂ is -NR'₃R'₄ or -OR'₃, wherein R'₃ and R'₄ are independently selected from the group consisting of H, substituted or unsubstituted C₁-C₂₄ alkyl, substituted or unsubstituted C₂-C₂₄ alkenyl, substituted or unsubstituted C₂-C₂₄ alkynyl, substituted or unsubstituted C₃-C₁₀ cycloalkyl, substituted or unsubstituted C₆-C₁₅ aryl and a substituted or unsubstituted heterocyclyl of 3-10 members, a substituted or unsubstituted heteroarylalkyl group with a ring having 3 to 10 members and an alkyl chain of 1 to 6 carbon atoms. More preferably, R'₃ and R'₄ are selected from the group consisting of H, methyl, ethyl, hexyl, dodecyl and hexadecyl. Even more preferably, R'₃ is H and R'₄ is selected from the group consisting of H, methyl, ethyl, hexyl, dodecyl and hexadecyl. According to an even more preferred embodiment, R"₂ is selected from -OH and -NH₂.

In another particular embodiment, R"₁ is selected from the group consisting of H, acetyl, lauroyl, myristoyl and palmitoyl, AA₁ is -L-Glu-, AA₂ is -L-Asp-, AA₃ is -L-Tyr-, AA₄ is -L-Tyr-, AA₅ is -L-Arg-, AA₆ is -L-Leu-, and R₂ is -NR'₃R'₄ or -OR'₃ wherein R'₃ and R'₄ are independently selected from the group consisting of H, methyl, ethyl, hexyl, dodecyl and hexadecyl, preferably R"₂ is -OH or -NH₂. More preferably, R"₁ is acetyl or palmitoyl and R"₂ is -NH₂. Even more preferably, p, r, s and t are 0.

In another particular embodiment, R"₁ is selected from the group consisting of H, acetyl, lauroyl, myristoyl and palmitoyl, AA₁ is -L-Pro-, AA₂ is -L-Asp-, AA₃ is -L-Tyr-, AA₄ is -L-Tyr-, AA₅ is -L-Lys-, AA₆ is -L-Leu-, and R₂ is -NR'₃R'₄ or -OR'₃ wherein R'₃ and R'₄ are independently selected from the group consisting of H, methyl, ethyl, hexyl, dodecyl and hexadecyl, preferably R"₂ is -OH or -NH₂. More preferably, R"₁ is acetyl or palmitoyl and R"₂ is -NH₂. Even more preferably, p, r, s and t are 0.

In another particular embodiment, R"₁ is selected from the group consisting of H, acetyl, lauroyl, myristoyl and palmitoyl, AA₁ is -L-Glu-, AA₂ is -L-Asp-, AA₃ is -L-Arg-, AA₄ is -L-Phe-, AA₅ is -L-Arg-, AA₆ is -L-Met-, and R₂ is -NR'₃R'₄ or -OR'₃ wherein R'₃ and R'₄ are independently selected from the group consisting of H, methyl, ethyl, hexyl, dodecyl and hexadecyl, preferably R"₂ is -OH or -NH₂. More preferably, R"₁ is acetyl or palmitoyl and R"₂ is -NH₂. Even more preferably, p, r, s and t are 0.

In another particular embodiment, R"₁ is selected from the group consisting of H, acetyl, lauroyl, myristoyl and palmitoyl, AA₁ is -L-Glu-, AA₂ is -L-Asp-, AA₃ is -L-Tyr-, AA₄ is -L-Tyr-, AA₅ is -L-Arg-, AA₆ is -L-Met-, and R₂ is -NR'₃R'₄ or -OR'₃ wherein R'₃ and R'₄ are independently selected from the group consisting of H, methyl, ethyl, hexyl, dodecyl or hexadecyl, preferably R"₂ is -OH or -NH₂. More preferably, R"₁ is acetyl or palmitoyl and R"₂ is -NH₂. Even more preferably, p, r, s and t are 0.

In another particular embodiment, R"₁ is selected from the group consisting of H, acetyl, lauroyl, myristoyl and palmitoyl, AA₁ is -L-Pro-, AA₂ is -L-Asp-, AA₃ is -L-Tyr-, AA₄ is -L-Tyr-, AA₅ is -L-Arg-, AA₆ is -L-Met-, and R₂ is -NR'₃R'₄ or -OR'₃ wherein R'₃ and R'₄ are independently selected from the group consisting of H, methyl, ethyl, hexyl, dodecyl and hexadecyl, preferably R"₂ is -OH or -NH₂. More preferably, R"₁ is acetyl or palmitoyl and R"₂ is -NH₂. Even more preferably, p, r, s and t are 0.

In another particular embodiment, R"₁ is selected from the group consisting of H, acetyl, lauroyl, myristoyl and palmitoyl, preferably R"₁ is selected from the group consisting of H, acetyl and palmitoyl, and R"₂ is selected from the group consisting of -OH or -NH₂.

In another particular embodiment, the peptide of general formula (IV) is selected from the group consisting of Ac-L-Glu-L-Asp-L-Tyr-L-Tyr-L-Arg-L-Leu-NH₂, Palm-L-Glu-L-Asp-L-Tyr-L-Tyr-L-Arg-L-Leu-NH₂, Ac-L-Pro-L-Asp-L-Tyr-L-Tyr-L-Lys-L-Leu-NH₂, Palm-L-Pro-L-Asp-L-Tyr-L-Tyr-L-Lys-L-Leu-NH₂, Ac-L-Glu-L-Asp-L-Arg-L-Phe-L-Arg-L-Met-NH₂, Palm-L-Glu-L-Asp-L-Arg-L-Phe-L-Arg-L-Met-NH₂, Ac-L-Glu-L-Asp-L-Tyr-L-Tyr-L-Arg-L-Met-NH₂, Palm-L-Glu-L-Asp-L-Tyr-L-Tyr-L-Arg-L-Met-NH₂, Ac-L-Pro-L-Asp-L-Tyr-L-Tyr-L-Arg-L-Met-NH₂ and Palm-L-Pro-L-Asp-L-Tyr-L-Tyr-L-Arg-L-Met-NH₂.

### BRIEF DESCRIPTION OF THE DRAWINGS

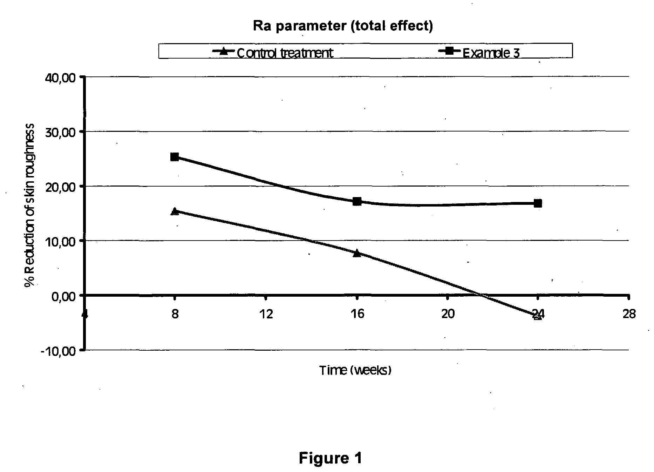

**Figure 1** shows **Ra** values for the mean of the different studied regions as function of time (total effect, mean of the total effects in periorbital and frontal region). It shows the comparison effect of control treatment (treatment with Botulinum toxin type A and placebo composition of Example 1) versus the treatment with Botulinum toxin type A and the composition of Example 3.

### EXAMPLES

The following specific examples given here are intended to illustrate the nature of this invention. These examples are for illustrative purposes only and should not be construed as limitations on the invention claimed herein.

### EXAMPLE 1. Preparation of a placebo composition for the in vivo studies.

| INGREDIENT (INCI Nomenclature) | | % IN WEIGHT |
|---|---|---|
| A | WATER (AQUA) | q.s.p. 100 |
| | DISODIUM EDTA | 0.3 |
| | PHENOXYETHANOL, METHYLPARABEN, ETHYLPARABEN,BUTYLPARABEN, PROPYLPARABEN, ISOBUTYLPARABEN | 0.7 |
| B | WATER (AQUA), POLYACRYLAMIDE, C13-14 ISOPARAFFIN, LAURETH-7 | 1 |
| C | CYCLOPENTASILOXANE, DIMETHICONE/VINYLDIMETHICONE | |
| | CROSSPOLYMER | 4 |
| | PEG/PPG-18/18 DIMETHICONE | 2.5 |
| D | ETHYLHEXYL METHOXYCINNAMATE | 3 |
| | BUTYL METHOXYDIBENZOYLMETHANE | 0.5 |
| | 4-METHYLBENZYLIDENE CAMPHOR | 0.5 |
| E | FRAGRANCE (PARFUM) | 0.2 |
| F | TRIETHANOLAMINE | q.s. |

Phase A ingredients were mixed, phase B was added and the mixture was homogenized. Phase C was added onto phase A+B while stirring until its total incorporation. Ingredients of phase D were melted at 65°C and added onto the previous mixture under stirring. Finally, the perfume (phase E) was added and the mixture was homogenized. The pH of the mixture was adjusted with triethanolamine (phase F) when necessary (final pH: 5.5-6.5).

### EXAMPLE 2. Preparation of a composition comprising peptide Acetyl-SEQ ID No.11-NH₂.

| INGREDIENT (INCI Nomenclature) | | % IN WEIGHT |
|---|---|---|
| A | WATER (AQUA) | q.s.p. 100 |
| | DISODIUM EDTA | 0.3 |
| | PHENOXYETHANOL, METHYLPARABEN, ETHYLPARABEN,BUTYLPARABEN, PROPYLPARABEN, ISOBUTYLPARABEN | 0.7 |
| B | WATER (AQUA), POLYACRYLAMIDE, C13-14 ISOPARAFFIN, LAURETH-7 | 3.5 |
| C | CYCLOPENTASILOXANE, DIMETHICONE/VINYLDIMETHICONE CROSSPOLYMER | 4 |
| | PEG/PPG-18/18 DIMETHICONE | 2.5 |
| D | ETHYLHEXYL METHOXYCINNAMATE | 3 |
| | BUTYL METHOXYDIBENZOYLMETHANE | 0.5 |
| | 4-METHYLBENZYLIDENE CAMPHOR | 0.5 |
| E | Ac-L-Glu-L-Glu-L-Met-L-Gln-L-Arg-L-Arg-NH₂ *(Acetyl-SEQ ID No. 11-NH₂)* | 0.005 |
| | PHENOXYETHANOL, METHYLPARABEN, ETHYLPARABEN,BUTYLPARABEN, PROPYLPARABEN, ISOBUTYLPARABEN | 0.03 |
| | WATER (AQUA) | 9.97 |
| F | FRAGRANCE (PARFUM) | 0.2 |
| G | TRIETHANOLAMINE | q.s. |

Phase A ingredients were mixed, phase B was added and the mixture was homogenized. Phase C was added onto phase A+B while stirring until its total incorporation. Ingredients of phase D were melted at 65°C and added onto the previous mixture under stirring. Phase E was added and the mixture was homogenized. Finally, the perfume (phase F) was added and the mixture was homogenized. The pH of the mixture was adjusted with triethanolamine (phase G) when necessary (final pH: 5.5-6.5).

### EXAMPLE 3. Preparation of a composition comprising peptide Acetyl-SEQ ID No.11-NH₂ and peptide H-L-Tyr-D-Ala-L-Gly-L-Phe-L-Leu-OH (SEQ ID No.35).

| INGREDIENT (INCI Nomenclature) | | % IN WEIGHT |
|---|---|---|
| A | WATER (AQUA) | q.s.p. 100 |
| | DISODIUM EDTA | 0.3 |
| | PHENOXYETHANOL, METHYLPARABEN, ETHYLPARABEN,BUTYLPARABEN, PROPYLPARABEN, ISOBUTYLPARABEN | 0.7 |
| B | WATER (AQUA), POLYACRYLAMIDE, C13-14 ISOPARAFFIN, LAURETH-7 | 1 |
| C | CYCLOPENTASILOXANE, DIMETHICONE/VINYLDIMETHICONE CROSSPOLYMER | 4 |
| | PEG/PPG-18/18 DIMETHICONE | 2.5 |
| D | ETHYLHEXYL METHOXYCINNAMATE | 3 |
| | BUTYL METHOXYDIBENZOYLMETHANE | 0.5 |
| | 4-METHYLBENZYLIDENE CAMPHOR | 0.5 |
| E | Ac-L-Glu-L-Glu-L-Met-L-Gln-L-Arg-L-Arg-NH₂ *(Acetyl-SEQ ID No. 11-NH₂)* | 0.005 |
| | PHENOXYETHANOL, METHYLPARABEN, ETHYLPARABEN,BUTYLPARABEN, PROPYLPARABEN, ISOBUTYLPARABEN | 0.03 |
| | WATER (AQUA) | 9.97 |
| F | H-L-Tyr-D-Ala-L-Gly-L-Phe-L-Leu-OH (*SEQ ID No.35)* | 0.0025 |
| | GLYCERIN | 0.5 |
| | CAPRYLYL GLYCOL | 0.025 |
| | WATER (AQUA) | 4.47 |
| G | FRAGRANCE (PARFUM) | 0.2 |
| H | TRIETHANOLAMINE | q.s. |

Phase A ingredients were mixed, phase B was added and the mixture was homogenized. Phase C was added onto phase A+B while stirring until its total incorporation. Ingredients of phase D were melted at 65°C and added onto the previous mixture under stirring. Phases E and F were added and the mixture was homogenized. Finally, the perfume (phase G) was added and the mixture was homogenized. The pH of the mixture was adjusted with triethanolamine (phase H) when necessary (final pH: 5.5-6.5).

### EXAMPLE 4. Preparation of a composition comprising peptide Acetyl-SEQ ID No.11-NH₂ and peptide H-L-Tyr-D-Ala-L-Gly-L-Phe-L-Leu-OH (SEQ ID No.35).

| INGREDIENT (INCI Nomenclature) | | % IN WEIGHT |
|---|---|---|
| A | LECITHIN | 0.40 |
| B | Ac-L-Glu-L-Glu-L-Met-L-Gln-L-Arg-L-Arg-NH₂ *(Acetyl-SEQ ID No.11-NH₂)* | 0.0025 |
| | PHENOXYETHANOL, METHYLPARABEN, ETHYLPARABEN,BUTYLPARABEN, PROPYLPARABEN, ISOBUTYLPARABEN | 0.015 |
| | WATER (AQUA) | 4.98 |
| C | H-L-Tyr-D-Ala-L-Gly-L-Phe-L-Leu-OH (*SEQ ID No.35)* | 0.0025 |
| | GLYCERIN | 0.5 |
| | CAPRYLYL GLYCOL | 0.025 |
| | WATER (AQUA) | 4.47 |
| D | WATER (AQUA) | q.s.p. 100 |
| | GLYCERIN | 2.38 |
| | PHENOXYETHANOL, METHYLPARABEN, ETHYLPARABEN,BUTYLPARABEN, PROPYLPARABEN, ISOBUTYLPARABEN | 0.47 |
| | CARBOMER | 0.15 |
| E | MINERAL OIL | 7.96 |
| | STEARIC ACID, PALMITIC ACID | 2.38 |
| | CETEARYL ALCOHOL | 1.59 |
| | DIMETHICONE | 0.15 |
| | BEESWAX (CERA ALBA) | 0.79 |
| F | TRIETHANOLAMINE | 0.88 |

Phases A, B and C ingredients were dissolved under stirring. In a separate vessel, phase D ingredients were heated up to melt. Then phase E ingredients were added and mixed up with phase D. The whole mixture was stirred with a turbine and phases A+B+C ingredients were added. The pH of the mixture was adjusted to 6.0-7.0 with triethanolamine (phase F) when necessary.

### EXAMPLE 5. Preparation of a composition comprising peptide Acetyl-SEQ ID No.11-NH₂ and peptide SEQ ID No.33.

| INGREDIENT (INCI Nomenclature) | | % IN WEIGHT |
|---|---|---|
| A | LECITHIN | 1.5 |
| | LECITHIN, GLYCOLIPIDS | 0.012 |
| | C24-28 ALKYL METHICONE | 1.9 |
| | PHENOXYETHANOL, METHYLPARABEN, ETHYLPARABEN,BUTYLPARABEN, | |
| | PROPYLPARABEN, ISOBUTYLPARABEN | 0.23 |
| | ETHYLHEXYL COCOATE | 15.1 |
| B | Ac-L-Glu-L-Glu-L-Met-L-Gln-L-Arg-L-Arg-NH₂ *(Acetyl-SEQ ID No.11-NH₂)* | 0.0024 |
| | H-L-Tyr-L-Gly-L-Gly-L-Phe-L-Leu-OH (*SEQ ID No.33)* | 0.0024 |
| | WATER (AQUA) | 0.24 |
| C | CYCLOPENTASILOXANE, DIMETHICONE CROSSPOLYMER | 52 |
| | CYCLOPENTASILOXANE | 28.9 |
| D | FRAGANCE (PARFUM) | 0.1 |
| E | Cl 17200 (RED 33) | 0.03 |

Phase A ingredients were heated at about 75-80°C. In a separate vessel, phase B ingredients were mixed, stirred until complete dissolution and then added into phase A and mixed together. The mixture was kept at 60°C.

Separately, phase C ingredients were mixed up and stirred until the dispersion of the silicones was completed. Then phase A+B was added to the mixture of phase C ingredients. Finally, the fragrance and colorant (phase D and E) were added.

### EXAMPLE 6. Preparation of a composition comprising peptide Acetyl-SEQ ID No.11-NH₂ and peptide SEQ ID No.33.

| INGREDIENT (INCI Nomenclature) | | % IN WEIGHT |
|---|---|---|
| A | ETHYLHEXYL COCOATE | 15.10 |
| | LECITHIN | 1.52 |
| | C24-28 ALKYL METHICONE | 1.9 |
| | LECITHIN, GLYCOLIPIDS | 0.0114 |
| | PHENOXYETHANOL, METHYLPARABEN, ETHYLPARABEN,BUTYLPARABEN, PROPYLPARABEN, ISOBUTYLPARABEN | 0.23 |
| B | Ac-L-Glu-L-Glu-L-Met-L-Gln-L-Arg-L-Arg-NH₂ *(Acetyl-SEQ ID No.11-NH₂)* | 0.0024 |
| | H-L-Tyr-L-Gly-L-Gly-L-Phe-L-Leu-OH (*SEQ ID No.33)* | 0.0024 |
| | WATER (AQUA) | 0.228 |
| C | CYCLOPENTASILOXANE, DIMETHICONE CROSSPOLYMER | 45.9 |
| D | CYCLOPENTASILOXANE | 35 |
| E | FRAGANCE (PARFUM) | 0.1 |
| F | Cl 17200 (RED 33) | 0.0003 |

Phase A ingredients were heated at about 75-80°C until they melt. In a separate vessel, phase B ingredients were dissolved at a temperature of about 75-80°C. Phase B was added into phase A under stirring, and the mixture was kept at 60°C.

Separately, phases C and D were mixed up and added to the heated mixture of phases A+B. Finally, the fragrance and colorant (phase E and F) were added.

### EXAMPLE 7. Preparation of a composition comprising peptide Acetyl-SEQ ID No.8-NH₂ and peptide H-L-Tyr-D-Ala-L-Gly-L-Phe-L-Leu-OH (SEQ ID No.35).

| INGREDIENT (INCI Nomenclature) | | % IN WEIGHT |
|---|---|---|
| A | WATER (AQUA) | q.s.p. 100 |
| | DISODIUM EDTA | 0.3 |
| | PHENOXYETHANOL, METHYLPARABEN, ETHYLPARABEN,BUTYLPARABEN, PROPYLPARABEN, ISOBUTYLPARABEN | 0.7 |
| B | WATER (AQUA), POLYACRYLAMIDE, C13-14 ISOPARAFFIN, LAURETH-7 | 1 |
| C | CYCLOPENTASILOXANE, | |
| | DIMETHICONE/VINYLDIMETHICONE CROSSPOLYMER | 4 |
| | PEG/PPG-18/18 DIMETHICONE | 2.5 |
| D | ETHYLHEXYL METHOXYCINNAMATE | 3 |
| | BUTYL METHOXYDIBENZOYLMETHANE | 0.5 |
| | 4-METHYLBENZYLIDENE CAMPHOR | 0.5 |
| E | Ac-L-Glu-L-Glu-L-Met-L-Gln-L-Arg-L-Arg-L-Ala-L-Asp-NH₂ *(Acetyl-SEQ ID No.8-NH₂)* | 0.005 |
| | PHENOXYETHANOL, METHYLPARABEN, ETHYLPARABEN,BUTYLPARABEN, PROPYLPARABEN, ISOBUTYLPARABEN | 0.03 |
| | WATER (AQUA) | 9.97 |
| F | H-L-Tyr-D-Ala-L-Gly-L-Phe-L-Leu-OH (*SEQ ID No.35)* | 0.0025 |
| | GLYCERIN | 0.5 |
| | CAPRYLYL GLYCOL | 0.025 |
| | WATER (AQUA) | 4.47 |
| G | FRAGRANCE (PARFUM) | 0.2 |
| H | TRIETHANOLAMINE | q.s. |

Phase A ingredients were mixed, phase B was added and the mixture was homogenized. Phase C was added onto phase A+B while stirring until its total incorporation. Ingredients of phase D were melted at 65°C and added onto the previous mixture under stirring. Phases E and F were added and the mixture was homogenized. Finally, the perfume (phase G) was added and the mixture was homogenized. The pH of the mixture was adjusted with triethanolamine (phase H) when necessary (final pH: 5.5-6.5).

### EXAMPLE 8. Preparation of a composition comprising peptide Acetyl-SEQ ID No.11-NH₂ and peptide H-L-Tyr-D-Ala-L-Gly-L-Phe-L-Leu-OH (SEQ ID No.35).

| INGREDIENT (INCI Nomenclature) | | % IN WEIGHT |
|---|---|---|
| A | WATER (AQUA) | q.s.p. 100 |
| | GLYCERIN | 5 |
| | PHENOXYETHANOL, METHYLPARABEN, ETHYLPARABEN,BUTYLPARABEN, | |
| | PROPYLPARABEN, ISOBUTYLPARABEN | 0.32 |
| | DISODIUM EDTA | 0.15 |
| | PROPYLENE GLYCOL | 5 |
| B | Ac-L-Glu-L-Glu-L-Met-L-Gln-L-Arg-L-Arg-NH₂ *(Acetyl-SEQ ID No.11-NH₂)* | 0.0025 |
| | PHENOXYETHANOL, METHYLPARABEN, ETHYLPARABEN,BUTYLPARABEN, PROPYLPARABEN, ISOBUTYLPARABEN | 0.015 |
| | WATER (AQUA) | 4.98 |
| C | H-L-Tyr-D-Ala-L-Gly-L-Phe-L-Leu-OH (*SEQ ID No.35)* | 0.0025 |
| | GLYCERIN | 0.5 |
| | CAPRYLYL GLYCOL | 0.025 |
| | WATER (AQUA) | 4.47 |
| D | POLYACRYLAMIDE, C13-14 ISOPARAFFIN, LAURETH-7 | 1 |
| | MINERAL OIL (PARAFFINUM LIQUIDUM) | 4 |
| | C24-28 ALKYL METHICONE | 0.5 |
| E | FRAGANCE (PARFUM) | 0.1 |
| F | WATER (AQUA) | 19.3 |
| | PHENOXYETHANOL, METHYLPARABEN, ETHYLPARABEN,BUTYLPARABEN, PROPYLPARABEN, ISOBUTYLPARABEN | 0.1 |
| | ACRYLATES/C10-30 ALKYL ACRYLATE CROSSPOLYMER | 0.6 |
| G | TRIETHANOLAMINE | 0.625 |

Phases A, B and C ingredients were dissolved. In a separate vessel, phase D ingredients were heated at about 80°C and then added into phases A+B+C and mixed together. Phases E and F were added under stirring. Finally, the pH of the mixture was adjusted to 6.0-7.0 with phase G.

### EXAMPLE 9. Preparation of a composition comprising peptide Acetyl-SEQ ID No.8-NH₂ and peptide H-L-Tyr-D-Ala-L-Gly-L-Phe-L-Leu-OH (SEQ ID No.35).

| INGREDIENT (INCI Nomenclature) | | % IN WEIGHT |
|---|---|---|
| A | WATER (AQUA) | q.s.p. 100 |
| | PROPYLENE GLYCOL | 5 |
| | GLYCERIN | 5 |
| | SORBITOL | 2 |
| | CYCLOPENTASILOXANE | 2 |
| | HYDROXYETHYLCELLULOSE | 1 |
| | XANTHAN GUM | 0.4 |
| | PHENOXYETHANOL, METHYLPARABEN, ETHYLPARABEN,BUTYLPARABEN, PROPYLPARABEN, ISOBUTYLPARABEN | 0.3 |
| | GUAR GUM | 0.1 |
| | DISODIUM EDTA | 0.15 |
| B | Ac-L-Glu-L-Glu-L-Met-L-Gln-L-Arg-L-Arg-L-Ala-L-Asp-NH₂ *(Acetyl-SEQ ID No.8-NH₂)* | 0.0025 |
| | PHENOXYETHANOL, METHYLPARABEN, ETHYLPARABEN,BUTYLPARABEN, PROPYLPARABEN, ISOBUTYLPARABEN | 0.015 |
| | WATER (AQUA) | 4.98 |
| C | H-L-Tyr-D-Ala-L-Gly-L-Phe-L-Leu-OH (*SEQ ID No.35)* | 0.0005 |
| | GLYCERIN | 0.1 |
| | CAPRYLYL GLYCOL | 0.005 |
| | WATER (AQUA) | 0.89 |
| D | WATER (AQUA) | 4.821 |
| | XANTHAN GUM | 0.0852 |
| | PECTIN | 0.0053 |
| | HYDROLYZED VEGETABLE PROTEIN | 0.0405 |
| | SERINE | 0.0025 |
| | ARGININE | 0.0025 |
| | PROLINE | 0.0025 |
| | PHENOXYETHANOL, METHYLPARABEN, ETHYLPARABEN,BUTYLPARABEN, PROPYLPARABEN, ISOBUTYLPARABEN | 0.0252 |
| | DISODIUM EDTA | 0.0153 |
| E | WATER (AQUA) | 2.3420 |
| | TOCOPHERYL ACETATE | 0.18 |
| | RETINYL PALMITATE | 0.18 |
| | PHOSPHOLIPIDS | 0.12 |
| | HYDROGENATED LECITHIN | 0.12 |
| | CARRAGEENANS (CHONDRUS CRISPUS) | 0.0015 |
| | CARBOMER | 0.0086 |
| | TRIETHANOLAMINE | 0.0067 |
| | ALCOHOL DENAT | 0.015 |
| | TOCOPHEROL | 0.0022 |
| | DISODIUM EDTA | 0.009 |
| | PHENOXYETHANOL, METHYLPARABEN, ETHYLPARABEN,BUTYLPARABEN, PROPYLPARABEN, ISOBUTYLPARABEN | 0.015 |
| F | WATER (AQUA) | 1.7919 |
| | GLYCERYL LINOLEATE, GLYCERYL LINOLENATE | 0.1 |
| | PHOSPHOLIPIDS | 0.08 |
| | CARRAGEENANS (CHONDRUS CRISPUS) | 0.001 |
| | CARBOMER | 0.0061 |
| | TRIETHANOLAMINE | 0.0048 |
| | DISODIUM EDTA | 0.0061 |
| | PHENOXYETHANOL, METHYLPARABEN, ETHYLPARABEN,BUTYLPARABEN PROPYLPARABEN, ISOBUTYLPARABEN | 0.0101 |
| G | FRAGANCE (PARFUM) | 0.15 |
| H | Cl 17200 (RED 33) | 0.0007 |
| E | TRIETHANOLAMINE | 0.08 |

Ingredients of phases A, B and C were mixed and homogenized. Phases D, E and F were successively added into phases A+B+C and mixed together. Phases G and H were added under stirring. Finally, the pH of the mixture was adjusted to 6.0-7.0 with phase E.

### EXAMPLE 10. Preparation of a composition comprising peptide Acetyl-SEQ ID No.11-NH₂, peptide H-L-Tyr-D-Ala-L-Gly-L-Phe-L-Leu-OH (SEQ ID No.35) and peptide Acetyl-SEQ ID No.39-NH₂.

| INGREDIENT (INCI Nomenclature) | | % IN WEIGHT |
|---|---|---|
| A | ETHYLHEXYL COCOATE | 15.10 |
| | LECITHIN | 1.52 |
| | C24-28 ALKYL METHICONE | 1.9 |
| | LECITHIN, GLYCOLIPIDS | 0.0114 |
| | PHENOXYETHANOL, METHYLPARABEN, ETHYLPARABEN,BUTYLPARABEN, PROPYLPARABEN, ISOBUTYLPARABEN | 0.23 |
| B | Ac-L-Glu-L-Glu-L-Met-L-Gln-L-Arg-L-Arg-NH₂ *(Acetyl-SEQ ID No. 11-NH₂)* | 0.0024 |
| | H-L-Tyr-D-Ala-L-Gly-L-Phe-L-Leu-OH (*SEQ ID No.35)* | 0.0024 |
| | Ac-L-Glu-L-Asp-L-Tyr-L-Tyr-L-Arg-L-Leu-NH₂ *(Acetyl-SEQ ID No.39-NH₂)* | 0.0024 |
| | WATER (AQUA) | 0.328 |
| C | CYCLOPENTASILOXANE, DIMETHICONE CROSSPOLYMER | 45.9 |
| D | CYCLOPENTASILOXANE | 34.9 |
| E | FRAGANCE (PARFUM) | 0.1 |
| F | Cl 17200 (RED 33) | 0.0003 |

Phase A ingredients were heated at about 75-80°C until they melt. In a separate vessel, phase B ingredients were dissolved at a temperature of about 75-80°C. Phase B was added into phase A under stirring, and the mixture was kept at 60°C.

Separately, phases C and D were mixed up and added to the heated mixture of phases A+B. Finally, the fragrance and colorant (phase E and F) were added.

### EXAMPLE 11. Preparation of a composition comprising Acetyl-SEQ ID No.4-NH₂ and peptide SEQ ID No.33.

| INGREDIENT (INCI Nomenclature) | | % IN WEIGHT |
|---|---|---|
| A | LECITHIN | 1.5 |
| | LECITHIN, GLYCOLIPIDS | 0.012 |
| | C24-28 ALKYL METHICONE | 1.9 |
| | PHENOXYETHANOL, METHYLPARABEN, ETHYLPARABEN,BUTYLPARABEN, PROPYLPARABEN, ISOBUTYLPARABEN | 0.23 |
| | ETHYLHEXYL COCOATE | 15.1 |
| B | Ac-L-Glu-L-Leu-L-Glu-L-Glu-L-Met-L-Gln-L-Arg--L-Arg-L-Ala-L-Asp-L-Gln-L-Leu-L-Ala-NH₂ | |
| | *(Acetyl-SEQ ID No.4-NH₂)* | 0.005 |
| | H-L-Tyr-L-Gly-L-Gly-L-Phe-L-Leu-OH (*SEQ ID No.33)* | 0.0025 |
| | WATER (AQUA) | 0.34 |
| C | CYCLOPENTASILOXANE, DIMETHICONE CROSSPOLYMER | 51.9 |
| | CYCLOPENTASILOXANE | 28.9 |
| D | FRAGANCE (PARFUM) | 0.1 |
| E | CI 17200 (RED 33) | 0.01 |

Phase A ingredients were heated at about 75-80°C. In a separate vessel, phase B ingredients were mixed, stirred until complete dissolution and then added into phase A and mixed together. The mixture was kept at 60°C.

Separately, phase C ingredients were mixed up and stirred until the dispersion of the silicones was completed. Then phase A+B was added to the mixture of phase C ingredients. Finally, the fragrance and colorant (phase D and E) were added.

### EXAMPLE 12. In vivo anti-wrinkle efficacy of combined treatment: Botulinum toxin type A and composition comprising peptide Acetyl-SEQ ID No.11-NH₂ and peptide H-L-Tyr-D-Ala-L-Gly-L-Phe-L-Leu-OH (SEQ ID No.35).

A monocentric study was performed in double blind conditions to evaluate the anti-wrinkle effect of the compositions of Examples 2 and 3 after Botulinum toxin type A injection. Treatment with Botulinum toxin type A and composition of Example 1 (placebo) was used as the control treatment. Profilometric analysis of silicon replica imprints was performed to analyze wrinkle depth 2, 4 and 6 months (2M, 4M and 6M) after Botulinum toxin type A injection.

The study was done on 30 Caucasian females with an average age of 51 years old. All subjects received Botulinum toxin type A injections (Vistabel®, Allergan) in each periorbital region (crow's feet, 12.5 Units, U) and frontal region (25 U), receiving a total of 50 U of toxin.

After injections, a group of 8 volunteers initiated the application of the composition of Example 2, another group of 9 volunteers received the composition of Example 3, and a final group of 13 volunteers received the placebo composition of Example 1. All volunteers applied the compositions of Examples 1, 2 or 3 twice a day.

Skin silicon replicas from the ocular periorbital region (crow's feet) and frontal region from 30 volunteers exposed to the treatments described before were obtained before Botulinum toxin type A injection (TO) and after 2, 4 and 6 months (2M, 4M and 6M) of the application of the placebo composition of Example 1 or the composition of Example 2 or Example 3. Briefly, on a clean Petri plate the silicon rubber material (Silflo®) was mixed with a catalyst in a ratio of about 3 drops of catalyst/ 2 ml paste. The two ingredients were mixed carefully together for 1 minute and were finally spread over the skin area in the periorbital and frontal regions, covering an area of 6 x 3 cm. After 12-15 minutes of drying, the silicone replica was peeled off from the skin.

The number of replicas per region obtained at each time point is indicated in Table 1:

**Table 1**

| **Treatment** | **Study region** | **Total subjects/region** | **Number of replicas/time** | | | |
|---|---|---|---|---|---|---|
| | | | **T0** | **2M** | **4M** | **6M** |
| **Example 2** | Frontal | 7 | 7 | 7 | - | - |
| | Periorbital | 8 | 8 | 8 | - | - |
| **Example 3** | Frontal | 9 | 9 | 9 | 9 | 4 |
| | Periorbital | 9 | 9 | 9 | 9 | 4 |
| **Control** | Frontal | 13 | 13 | 10 | 10 | 4 |
| | Periorbital | 12 | 12 | 9 | 9 | 4 |

Analysis of the study replicas was performed by confocal profilometry. A common area between replica obtained before treatment (T0 replica) and replicas at the different sampled times, from the same volunteer and study region (frontal or periorbital region) was selected for analysis. A confocal profilometer (PLµ, Industrial microscope Eclipse L150A, Nikon) mechanically scanned the replica surface by direct illumination and the reflected signal was observed with a CCD (Charged-Coupled Device) array and analyzed with different image data processing algorithm.

From each volunteer and each study region (frontal or periorbital), two independent previously selected areas (two wrinkles) were analyzed. The analyzed area was exactly the same area at all sampled times for the same study region and the same volunteer.

From the profilometric analysis of each macroroughness (or wrinkle) the profilometer software calculated the roughness parameters (**Ra**, Average surface roughness, the deviation from arithmetic mean of the evaluated relief; **RMS,** Root Mean Square roughness average of the evaluated relief; and **PV,** Peak-To-Valley distance, distance between the point of the maximum height and the point of the minimal height) values and processed the three-dimensional image. The **Ra** average is understood as the wrinkle reduction average when is related to wrinkle at T0. The Ra max is understood as the maximum of the wrinkle reduction average.

### Results

### CONTROL TREATMENT EFFECTS

Percentages of mean reduction of the roughness parameters at 2, 4, and 6 months (2M, 4M and 6M) after Botulinum toxin type A injection and the application of placebo composition of Example 1 treatment with respect to base line (TO) are shown in Table 2:

**Table 2**

| % **Reduction of roughness: total effect** (effect in periorbital and frontal region) | | | | | |
|---|---|---|---|---|---|
| **Sampled times** | **N** | **Cal**. | **PV** | **RMS** | **Ra** |
| **2M versus T0** | 11 | **Mean** | **10.52** | **15.24** | **15.42** |
| **4M versus T0** | 11 | **Mean** | **5.56** | **7.82** | **7.72** |
| **6M versus T0** | 4 | **Mean** | **0.24** | **-2.93** | **-3.76** |

Figure 1 shows **Ra** values for the mean of the different studied regions as function of time (total effect, mean of the effects in periorbital and frontal region). For the control treatment, the anti-wrinkle effect after 4 months was only 7.72% and the anti-wrinkle effect was completey lost 6 months after Botulinum toxin type A injection. The maximum of the wrinkle reduction average (Ra max 38.57%) for the control group was observed during the first month after the administration of Botulinum toxin type A and application of the placebo composition of Example 1.

### TREATMENT EFFECTS OF THE COMPOSITION OF EXAMPLE 2

Percentages of mean reduction of the roughness parameters at 2 months (2M) after Botulinum toxin type A injection and the application of the composition of Example 2 with respect to base line (TO) are shown in Table 3:

**Table 3**

| **% Reduction of roughness: total effect** (effect in periorbital and frontal region) | | | | | |
|---|---|---|---|---|---|
| **Sampled times** | **N** | **Cal**. | **PV** | **RMS** | **Ra** |
| **2M versus T0** | 9 | **Mean** | **20.28** | **27.68** | **27.87** |

When Botulinum toxin type A injection was combined with the treatment with the composition of Example 2, the anti-wrinkle effect after 2 months was 27.87% (almost double of that obtained with the control treatment at the same time). The wrinkle reduction average (Ra) after 2 months for the group of treatment comprising the composition of Example 2 was 72.3% of the maximum of the wrinkle reduction average (Ra max) of the control treatment, i.e. Botulinum toxin type A injection and placebo composition.

### TREATMENT EFFECTS OF THE COMPOSITION OF EXAMPLE 3

Percentages of mean reduction of the roughness parameters at 2, 4, and 6 months (2M, 4M and 6M) after Botulinum toxin type A injection and the application of the composition of Example 3 versus base line (TO) are shown in Table 4:

**Table 4**

| **% Reduction of roughness: total effect** (effect in periorbital and frontal region) | | | | | |
|---|---|---|---|---|---|
| **Sampled times** | **N** | **Cal**. | **PV** | **RMS** | **Ra** |
| **2M versus T0** | 10 | **Mean** | **20.38** | **24.76** | **25.31** |
| **4M versus T0** | 10 | **Mean** | **11.07** | **16.36** | **17.11** |
| **6M versus T0** | 4 | **Mean** | **18.08** | **17.41** | **16.77** |

When Botulinum toxin type A injection was combined with the application of the composition of Example 3, the anti-wrinkle effect was 16.77% even 6 months after the Botulinum toxin type A injection and application of the composition of Example 3.

The wrinkle reduction average (Ra) after 2 months for the group of treatment comprising the composition of Example 3 was 65.6% of the maximum of the wrinkle reduction average (Ra max) of the control treatment, 44.4% of Ra max after 4 months and 43.5% of Ra max after 6 months.

### TREATMENT COMPRISING THE COMPOSITION OF EXAMPLE 3 VERSUS CONTROL TREATMENT

Figure 1 shows the comparison effect of the control treatment versus the treatment with the composition of Example 3, after Botulinum toxin type A injection in both cases. Treatment with Botulinum toxin type A and the composition of Example 3 presented a better anti-wrinkle effect than the effect observed in the case of the control treatment.

## Claims

1. A non-therapeutic method for skin antiaging treatment which prolongs in time the effects of treatment with Botulinum toxin injections, the method comprising:
a. The administration of an effective amount of Botulinum toxin to an area of facial and/or neck skin,
b. and the administration, from once a week to ten times a day, of a cosmetic composition comprising a cosmetically effective amount of at least one peptide selected from the group consisting of SEQ ID No.11, SEQ ID No.4, or a sequence of 7 to 12 adjacent amino acids contained in SEQ ID No.4, wherein said sequence comprises the amino acid sequence of SEQ ID No.11, and according to the general formula (I):
R₁-AA-R₂ (I)
its stereoisomers, mixtures thereof, and/or its cosmetically acceptable salts thereof, in which AA is a sequence selected from the group consisting of SEQ ID No.11, SEQ ID No.4, or a sequence of 7 to 12 adjacent amino acids contained in SEQ ID No.4, wherein said sequence comprises the amino acid sequence of SEQ ID No.11;
wherein:
R₁ is selected from the group consisting of H, substituted or non-substituted non-cyclic aliphatic group, substituted or non-substituted alicyclyl, substituted or non-substituted heterocyclyl, substituted or non-substituted heteroarylalkyl, substituted or non-substituted aryl, substituted or non-substituted aralkyl and R₅-C(O)-; and
R₂ is selected from the group consisting of -NR₃R₄, -OR₃ and -SR₃; where R₃ and R₄ are independently selected from the group consisting of H, substituted or non-substituted non-cyclic aliphatic group, substituted or non-substituted alicyclyl, substituted or non-substituted heterocyclyl, substituted or non-substituted heteroarylalkyl, substituted or non-substituted aryl and substituted or non-substituted aralkyl;
wherein R₅ is selected from the group consisting of H, substituted or non-substituted non-cyclic aliphatic group, substituted or non-substituted alicyclyl, substituted or non-substituted aryl, substituted or non-substituted aralkyl, substituted or non-substituted heterocyclyl and substituted or non-substituted heteroarylalkyl;
and at least one cosmetically acceptable excipient or adjuvant.

2. The method of claim 1, wherein the skin antiaging treatment is a treatment for reducing or eliminating wrinkles of the facial and/or neck skin.

3. The method of claim 1, wherein R₁ is selected from the group consisting of H, a polymer of general formula (III) 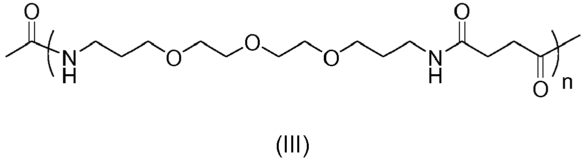 wherein n ranges between 1 to 100, and R₅-CO-, wherein R₅ is selected from the group consisting of substituted or unsubstituted C₁-C₂₄ alkyl radical, substituted or unsubstituted C₂-C₂₄ alkenyl radical, substituted or unsubstituted C₂-C₂₄ alkynyl radical, substituted or unsubstituted C₃-C₂₄ cycloalkyl radical, substituted or unsubstituted C₅-C₂₄ cycloalkenyl radical, substituted or unsubstituted C₈-C₂₄ cycloalkynyl radical, substituted or unsubstituted C₆-C₃₀ aryl radical, substituted or unsubstituted C₇-C₂₄ aralkyl radical, a substituted or unsubstituted heterocyclyl radical having 3-10 ring members, a substituted or unsubstituted heteroarylalkyl radical having 2 to 24 carbon atoms and having 1 to 3 atoms other than carbon wherein the alkyl chain is of 1 to 6 carbon atoms.

4. The method claim 1, wherein R₃ and R₄ are independently selected from the group consisting of H, substituted or unsubstituted C₁-C₂₄ alkyl, substituted or unsubstituted C₂-C₂₄ alkenyl, substituted or unsubstituted C₂-C₂₄ alkynyl, substituted or unsubstituted C₃-C₂₄ cycloalkyl, substituted or unsubstituted C₅-C₂₄ cycloalkenyl, substituted or unsubstituted C₈-C₂₄ cycloalkynyl, substituted or unsubstituted C₆-C₃₀ aryl, substituted or unsubstituted C₇-C₂₄ aralkyl, a substituted or unsubstituted heterocyclyl having 3-10 ring members, and a substituted or unsubstituted heteroarylalkyl group having 2 to 24 carbon atoms and having 1 to 3 atoms other than carbon wherein the alkyl chain is of 1 to 6 carbon atoms and a polymer of general formula (III) wherein n ranges between 1 and 100.

5. The method of claim 1, wherein AA is a sequence of adjacent amino acids selected from the group consisting of SEQ ID No.4, SEQ ID No.8, SEQ ID No.11, SEQ ID No.14, SEQ ID No.15, SEQ ID No 16, SEQ ID No.17, SEQ ID No.18, SEQ ID No.19, SEQ ID No.20, SEQ ID No.21, SEQ ID No.22, SEQ ID No.23, SEQ ID No.24, SEQ ID No.25 and SEQ ID No.26.

6. The method of claim 1, wherein the cosmetic composition further contains a cosmetically effective amount of at least one enkephalin-derived peptide of general formula (II): 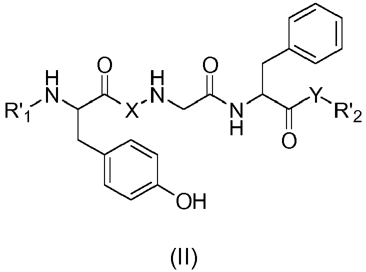 its stereoisomers, mixtures thereof, and/or its cosmetically acceptable salts thereof,
wherein:
X and Y are independently selected from the group consisting of natural amino acids and non-natural amino acids;
R'₁ is selected from the group consisting of H, substituted or non-substituted non-cyclic aliphatic group, substituted or non-substituted alicyclyl, substituted or non-substituted heterocyclyl, substituted or non-substituted heteroarylalkyl, substituted or non-substituted aryl, substituted or non-substituted aralkyl and R₅-C(O)-; and
R'₂ is selected from the group consisting of -NR₃R₄, -OR₃ and -SR₃; where R₃ and R₄ are independently selected from the group consisting of H, substituted or non-substituted non-cyclic aliphatic group, substituted or non-substituted alicyclyl, substituted or non-substituted heterocyclyl, substituted or non-substituted heteroarylalkyl, substituted or non-substituted aryl and substituted or non-substituted aralkyl;
wherein R₅ is selected from the group consisting of H, substituted or non-substituted non-cyclic aliphatic group, substituted or non-substituted alicyclyl, substituted or non-substituted aryl, substituted or non-substituted aralkyl, substituted or non-substituted heterocyclyl and substituted or non-substituted heteroarylalkyl.

7. The method of claim 6, wherein the sequence of adjacent amino acids contained in the general formula (II) is a sequence selected from the group consisting of SEQ ID No.33, SEQ ID No.34, SEQ ID No.35, SEQ ID No.36, SEQ ID No.37 and SEQ ID No.38.

8. The method of claim 1, wherein the cosmetic composition further contains a cosmetically effective amount of at least one peptide of general formula (IV):
R"₁-Aₚ-Bᵣ-AA₁-AA₂-AA₃-AA₄-AA₅-AA₆-Cₛ-Dₜ-R"₂ (IV)
its stereoisomers, mixtures thereof, and/or its cosmetically acceptable salts thereof, wherein:
AA₁ is selected from the group consisting of -Asp-, -Glu- and -Pro-;
AA₂ is -Asp-;
AA₃ is selected from the group consisting of -Tyr- and -Arg-;
AA₄ is selected from the group consisting of -Phe- and -Tyr-;
AA₅ is selected from the group consisting of -Arg- and -Lys-;
AA₆ is selected from the group consisting of -Leu- and -Met-;
A, B, C and D are independently selected from the group consisting of natural amino acids and non-natural amino acids;
p, r, s and t are independently selected and range between 0 and 1;
R"₁ is selected from the group consisting of H, substituted or non-substituted non-cyclic aliphatic group, substituted or non-substituted alicyclyl, substituted or non-substituted heterocyclyl, substituted or non-substituted heteroarylalkyl, substituted or non-substituted aryl, substituted or non-substituted aralkyl and R'₅-C(O)-; and
R"₂ is selected from the group consisting of -NR'₃R'₄, -OR'₃ and -SR'₃; where R'₃ and R'₄ are independently selected from the group consisting of H, substituted or non-substituted non-cyclic aliphatic group, substituted or non-substituted alicyclyl, substituted or non-substituted heterocyclyl, substituted or non-substituted heteroarylalkyl, substituted or non-substituted aryl and substituted or non-substituted aralkyl;
wherein R'₅ is selected from the group consisting of H, substituted or non-substituted non-cyclic aliphatic group, substituted or non-substituted alicyclyl, substituted or non-substituted aryl, substituted or non-substituted aralkyl, substituted or non-substituted heterocyclyl and substituted or non-substituted heteroarylalkyl.

9. The method of claim 1, wherein the cosmetic composition is administered by topical, transdermal, enteral or parenteral route, adhesive or non-adhesive patches, oral, nasal or inhalational route or by intradermal, intramuscular, intravenous, intraperitoneal or subcutaneous injection, iontophoresis, sonophoresis, electroporation, mechanical pressure, osmotic pressure gradient, occlusive treatment, microinjections, pressure injections without needles or micro-electric patches.

10. The method of claim 1, wherein the peptides of general formula (I) and/or (II) are incorporated into a delivery or a sustained release system selected from the group consisting of liposomes, mixed liposomes, oleosomes, niosomes, miniparticles, milliparticles, microparticles, nanoparticles and solid lipid nanoparticles, nanostructured lipid carriers, sponges, cyclodextrins, vesicles, micelles, mixed micelles of surfactants, surfactant-phospholipid mixed micelles, millispheres, microspheres, nanospheres, lipospheres, millicapsules, microcapsules, nanocapsules, microemulsions and nanoemulsions.

11. The method of claim 1, wherein the cosmetic composition presents a formulation selected from the group consisting of creams, multiple emulsions, anhydrous compositions, aqueous dispersions, oils, milks, balsams, foams, lotions, gels, cream gels, hydroalcoholic solutions, hydroglycolic solutions, hydrogels, liniments, sera, soaps, shampoos, conditioners, serums, ointments, mousses, pomades, powders, bars, pencils, sprays and aerosols.

12. The method of claim 1, wherein the cosmetic composition is found incorporated into a product selected from the group consisting of capsules, vials, syringes, preloaded syringes, under-eye concealers, make-up foundation, make-up removal lotions, make-up removal milks, eye shadows, lipsticks, lip gloss, lip protectors and powders.

13. The method of claim 1, wherein the cosmetic composition is incorporated into a fabric, a non-woven fabric, garment or a medical device.

14. The method of claim 1, wherein the cosmetic composition further comprises a cosmetically effective amount of at least one active agent selected from the group of cyclic adenosine monophosphate synthesis stimulating agents, elastase inhibiting agents, matrix metalloproteinases inhibiting agents, melanin synthesis stimulating or inhibiting agents, whitening or depigmenting agents, propigmenting agents, self-tanning agents, antiaging agents, NO-synthase inhibiting agents, 5α-reductase inhibiting agents, lysyl- and/or prolyl hydroxylase inhibiting agents, antioxidants, free radical scavengers and/or agents against atmospheric pollution, reactive carbonyl species scavengers, anti-glycation agents, antihistamine agents, antiviral agents, antiparasitic agents, emulsifiers, emollients, organic solvents, liquid propellants, skin and/or hair conditioners, humectants, substances that retain moisture, alpha hydroxyacids, beta hydroxyacids, moisturizers, epidermal hydrolytic enzymes, vitamins, pigments or colorants, dyes, gelling polymers, thickeners, surfactants, softening agents, anti-wrinkle agents, agents able to reduce or treat the bags under the eyes, exfoliating agents, antimicrobial agents, antifungal agents, fungistatic agents, bactericidal agents, bacteriostatic agents, agents stimulating the synthesis of dermal or epidermal macromolecules and/or capable of inhibiting or preventing their degradation, collagen synthesis-stimulating agents, elastin synthesis-stimulating agents, decorin synthesis-stimulating agents, laminin synthesis-stimulating agents, defensin synthesis-stimulating agents, chaperone synthesis-stimulating agents, aquaporin synthesis-stimulating agents, hyaluronic acid synthesis-stimulating agents, fibronectin synthesis-stimulating agents, sirtuin synthesis-stimulating agents, agents stimulating the synthesis of lipids and components of the stratum corneum, agents stimulating the synthesis of ceramides, agents that inhibit collagen degradation, agents that inhibit elastin degradation, agents that inhibit serine proteases such us cathepsin G, agents stimulating fibroblast proliferation, agents stimulating keratinocyte proliferation, agents stimulating adipocyte proliferation, agents stimulating melanocyte proliferation, agents stimulating keratinocyte differentiation, agents stimulating adipocyte differentiation, agents that inhibit acetylcholinesterase, skin relaxant agents, agents that inhibit acetylcholine receptors aggregation, agents that inhibit muscle contraction, glycosaminoglycan synthesis-stimulating agents, antihyperkeratosis agents, comedolytic agents, antipsoriasis agents, DNA repair agents, DNA protecting agents, stabilizers, anti-itching agents, agents for the treatment and/or care of sensitive skin, firming agents, anti-stretch mark agents, binding agents, agents regulating sebum production, lipolytic agents or agents stimulating lipolysis, anti-cellulite agents, antiperspirant agents, agents stimulating healing, coadjuvant healing agents, agents stimulating reepithelialization, coadjuvant reepithelialization agents, cytokine growth factors, calming agents, anti-inflammatory agents, anesthetic agents, agents acting on capillary circulation and/or microcirculation, agents stimulating angiogenesis, agents that inhibit vascular permeability, venotonic agents, agents acting on cell metabolism, agents to improve dermal-epidermal junction, agents inducing hair growth, hair growth inhibiting or retardant agents, preservatives, perfumes, chelating agents, vegetable extracts, essential oils, marine extracts, agents obtained from a biofermentation process, mineral salts, cell extracts and sunscreens, organic or mineral photoprotective agents active against ultraviolet A and/or B rays, and/or mixtures thereof.

15. A kit for skin antiaging treatment according to claim 1, comprising:
a. Botulinum toxin,
b. and at least one cosmetic composition comprising a cosmetically effective amount of at least one peptide selected from the group consisting of SEQ ID No.11, SEQ ID No.4, or a sequence of 7 to 12 adjacent amino acids contained in SEQ ID No.4, wherein said sequence comprises the amino acid sequence of SEQ ID No.11, and according to the general formula (I):
R₁-AA-R₂ (I)
its stereoisomers, mixtures thereof, and/or its cosmetically acceptable salts thereof, in which AA is a sequence selected from the group consisting of SEQ ID No.11, SEQ ID No.4, or a sequence of 7 to 12 adjacent amino acids contained in SEQ ID No.4, wherein said sequence comprises the amino acid sequence of SEQ ID No.11;
wherein:
R₁ is selected from the group consisting of H, substituted or non-substituted non-cyclic aliphatic group, substituted or non-substituted alicyclyl, substituted or non-substituted heterocyclyl, substituted or non-substituted heteroarylalkyl, substituted or non-substituted aryl, substituted or non-substituted aralkyl and R₅-C(O)-; and
R₂ is selected from the group consisting of -NR₃R₄, -OR₃ and -SR₃; where R₃ and R₄ are independently selected from the group consisting of H, substituted or non-substituted non-cyclic aliphatic group, substituted or non-substituted alicyclyl, substituted or non-substituted heterocyclyl, substituted or non-substituted heteroarylalkyl, substituted or non-substituted aryl and substituted or non-substituted aralkyl;
wherein R₅ is selected from the group consisting of H, substituted or non-substituted non-cyclic aliphatic group, substituted or non-substituted alicyclyl, substituted or non-substituted aryl, substituted or non-substituted aralkyl, substituted or non-substituted heterocyclyl and substituted or non-substituted heteroarylalkyl;
and at least one cosmetically acceptable excipient or adjuvant.

16. A kit as in claim 15, wherein the cosmetic composition further contains a cosmetically effective amount of at least one enkephalin-derived peptide of general formula (II): 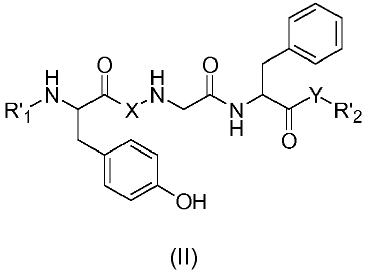 Its stereoisomers, mixtures thereof, and/or its cosmetically acceptable salts thereof,
wherein:
X and Y are independently selected from the group consisting of natural amino acids and non-natural amino acids;
R'₁ is selected from the group consisting of H, substituted or non-substituted non-cyclic aliphatic group, substituted or non-substituted alicyclyl, substituted or non-substituted heterocyclyl, substituted or non-substituted heteroarylalkyl, substituted or non-substituted aryl, substituted or non-substituted aralkyl and R₅-C(O)-; and
R'₂ is selected from the group consisting of -NR₃R₄, -OR₃ and -SR₃; where R₃ and R₄ are independently selected from the group consisting of H, substituted or non-substituted non-cyclic aliphatic group, substituted or non-substituted alicyclyl, substituted or non-substituted heterocyclyl, substituted or non-substituted heteroarylalkyl, substituted or non-substituted aryl and substituted or non-substituted aralkyl;
wherein R₅ is selected from the group consisting of H, substituted or non-substituted non-cyclic aliphatic group, substituted or non-substituted alicyclyl, substituted or non-substituted aryl, substituted or non-substituted aralkyl, substituted or non-substituted heterocyclyl and substituted or non-substituted heteroarylalkyl,
and/or a cosmetically effective amount of at least one peptide of general formula (IV):
R"₁-Aₚ-Bᵣ-AA₁-AA₂-AA₃-AA₄-AA₅-AA₆-Cₛ-DₜR"₂ (IV)
its stereoisomers, mixtures thereof, and/or its cosmetically acceptable salts thereof, wherein:
AA₁ is selected from the group consisting of -Asp-, -Glu- and -Pro-;
AA₂ is -Asp-;
AA₃ is selected from the group consisting of -Tyr- and -Arg-;
AA₄ is selected from the group consisting of -Phe- and -Tyr-;
AA₅ is selected from the group consisting of -Arg- and -Lys-;
AA₆ is selected from the group consisting of -Leu- and -Met-;
A, B, C and D are independently selected from the group consisting of natural amino acids and non-natural amino acids;
p, r, s and t are independently selected and range between 0 and 1;
R"₁ is selected from the group consisting of H, substituted or non-substituted non-cyclic aliphatic group, substituted or non-substituted alicyclyl, substituted or non-substituted heterocyclyl, substituted or non-substituted heteroarylalkyl, substituted or non-substituted aryl, substituted or non-substituted aralkyl and R'₅-C(O)-; and
R"₂ is selected from the group consisting of -NR'₃R'₄, -OR'₃ and -SR'₃; where R'₃ and R'₄ are independently selected from the group consisting of H, substituted or non-substituted non-cyclic aliphatic group, substituted or non-substituted alicyclyl, substituted or non-substituted heterocyclyl, substituted or non-substituted heteroarylalkyl, substituted or non-substituted aryl and substituted or non-substituted aralkyl;
wherein R'₅ is selected from the group consisting of H, substituted or non-substituted non-cyclic aliphatic group, substituted or non-substituted alicyclyl, substituted or non-substituted aryl, substituted or non-substituted aralkyl, substituted or non-substituted heterocyclyl and substituted or non-substituted heteroarylalkyl.

## Patentansprüche

1. Nichttherapeutisches Verfahren zur Haut-Anti-Aging-Behandlung, welches die Auswirkungen der Behandlung mit Botulinumtoxininjektionen zeitlich verlängert, wobei das Verfahren Folgendes umfasst:
a. Die Verabreichung einer wirksamen Menge an Botulinumtoxin auf einen Bereich der Gesichts- und/oder Halshaut,
b. und die Verabreichung von einmal wöchentlich bis zehnmal am Tag einer kosmetischen Zusammensetzung, die eine kosmetisch wirksame Menge von mindestens einem Peptid, das aus der Gruppe ausgewählt ist, die aus SEQ ID Nr. 11, SEQ ID Nr. 4 oder einer Sequenz von 7 bis 12 benachbarten Aminosäuren, die in SEQ ID Nr. 4 enthalten sind, besteht, wobei die Sequenz die Aminosäurensequenz von SEQ ID Nr. 11 umfasst, und gemäß der allgemeinen Formel (I):
R₁-AA-R₂ (I),
seinen Stereoisomeren, Mischungen davon und/oder seinen kosmetisch verträglichen Salzen davon, wobei AA eine Sequenz ist, die aus der Gruppe ausgewählt ist, die aus SEQ ID Nr. 11, SEQ ID Nr. 4 oder einer Sequenz von 7 bis 12 benachbarten Aminosäuren, die in SEQ ID Nr. 4 enthalten sind, besteht, wobei die Sequenz die Aminosäurensequenz von SEQ ID Nr. 11 umfasst;
wobei:
R₁ aus der Gruppe ausgewählt ist, die aus H, substituierte oder nicht substituierte nicht zyklische aliphatische Gruppe, substituiertes oder nicht substituiertes Alicyclyl, substituiertes oder nicht substituiertes Heterocyclyl, substituiertes oder nicht substituiertes Heteroarylalkyl, substituiertes oder nicht substituiertes Aryl, substituiertes oder nicht substituiertes Aralkyl und R₅-C(O)- besteht; und
R₂ aus der Gruppe ausgewählt ist, die aus -NR₃R₄, -OR₃ und -SR₃ besteht; wobei R₃ und R₄ unabhängig aus der Gruppe ausgewählt sind, die aus H, substituierte oder nicht substituierte nicht zyklische aliphatische Gruppe, substituiertes oder nicht substituiertes Alicyclyl, substituiertes oder nicht substituiertes Heterocyclyl, substituiertes oder nicht substituiertes Heteroarylalkyl, substituiertes oder nicht substituiertes Aryl und substituiertes oder nicht substituiertes Aralkyl besteht;
wobei R₅ aus der Gruppe ausgewählt ist, die aus H, substituierte oder nicht substituierte nicht zyklische aliphatische Gruppe, substituiertes oder nicht substituiertes Alicyclyl, substituiertes oder nicht substituiertes Aryl, substituiertes oder nicht substituiertes Aralkyl, substituiertes oder nicht substituiertes Heterocyclyl und substituiertes oder nicht substituiertes Heteroarylalkyl besteht;
und mindestens einen kosmetisch verträglichen Excipienten oder Adjuvans umfasst.

2. Verfahren nach Anspruch 1, wobei die Haut-Anti-Aging-Behandlung eine Behandlung zur Reduzierung oder Entfernung von Falten der Gesichts- und/oder Halshaut ist.

3. Verfahren nach Anspruch 1, wobei R₁ aus der Gruppe ausgewählt ist, die aus H, einem Polymer der allgemeinen Formel (III) 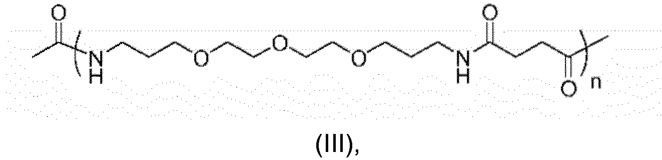 wobei n zwischen 1 und 100 beträgt, und R₅-CO- besteht, wobei R₅ aus der Gruppe ausgewählt ist, die aus substituiertes oder unsubstituiertes C₁-C₂₄-AlkylRadikal, substituiertes oder unsubstituiertes C₂-C₂₄-Alkenyl-Radikal, substituiertes oder unsubstituiertes C₂-C₂₄-Alkynyl-Radikal, substituiertes oder unsubstituiertes C₃-C₂₄-Cycloalykl-Radikal, substituiertes oder unsubstituiertes C₅-C₂₄-Cycloalkenyl-Radikal, substituiertes oder unsubstituiertes C₈-C₂₄-Cycloalkynyl-Radikal, substituiertes oder unsubstituiertes C₆-C₃₀-Aryl-Radikal, substituiertes oder unsubstituiertes C₇-C₂₄-Aralkyl-Radikal, einem substituierten oder unsubstituierten Heterocyclyl-Radikal, das 3-10 Ringelemente aufweist, einem substituierten oder unsubstituierten Heteroarylalkyl-Radikal, das 2 bis 24 Kohlenstoffatome aufweist und 1 bis 3 Atome aufweist, die sich von Kohlenstoff unterscheiden, wobei die Alkylkette 1 bis 6 Kohlenstoffatome beträgt, besteht.

4. Verfahren nach Anspruch 1, wobei R₃ und R₄ unabhängig aus der Gruppe ausgewählt sind, die aus H, substituiertes oder unsubstituiertes C₁-C₂₄-Alkyl, substituiertes oder unsubstituiertes C₂-C₂₄-Alkenyl, substituiertes oder unsubstituiertes C₂-C₂₄-Alkynyl, substituiertes oder unsubstituiertes C₃-C₂₄-Cycloalkyl, substituiertes oder unsubstituiertes C₅-C₂₄-Cycloalkenyl, substituiertes oder unsubstituiertes C₈-C₂₄-Cycloalkynyl, substituiertes oder unsubstituiertes C₆-C₃₀-Aryl, substituiertes oder unsubstituiertes C₇-C₂₄-Aralkyl, einem substituierten oder unsubstituierten Heterocyclyl, das 3-10 Ringelemente aufweist, und einer substituierten oder unsubstituierten Heteroarylalkylgruppe, die 2 bis 24 Kohlenstoffatome aufweist und 1 bis 3 Atome aufweist, die sich von Kohlenstoff unterscheiden, wobei die Alkylkette 1 bis 6 Kohlenstoffatome beträgt, und einem Polymer der allgemeinen Formel (III), wobei n zwischen 1 und 100 beträgt, besteht.

5. Verfahren nach Anspruch 1, wobei AA eine Sequenz von benachbarten Aminosäuren ist, ausgewählt aus der Gruppe, die aus SEQ ID Nr. 4, SEQ ID Nr. 8, SEQ ID Nr. 11, SEQ ID Nr. 14, SEQ ID Nr. 15, SEQ ID Nr. 16, SEQ ID Nr. 17, SEQ ID Nr. 18, SEQ ID Nr. 19, SEQ ID Nr. 20, SEQ ID Nr. 21, SEQ ID Nr. 22, SEQ ID Nr. 23, SEQ ID Nr. 24, SEQ ID Nr. 25 und SEQ ID Nr. 26 besteht.

6. Verfahren nach Anspruch 1, wobei die kosmetische Zusammensetzung ferner eine kosmetisch wirksame Menge von mindestens einem von Enkephalin abgeleiteten Peptid der folgenden allgemeinen Formel (II): 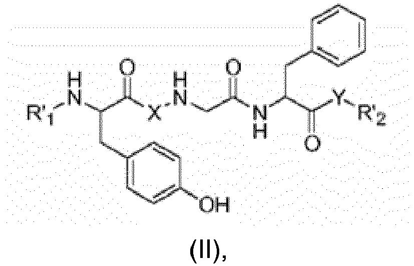 seinen Stereoisomeren, Mischungen davon und/oder seinen kosmetisch verträglichen Salzen davon enthält,
wobei:
X und Y unabhängig aus der Gruppe ausgewählt sind, die aus natürlichen Aminosäuren und nicht-natürlichen Aminosäuren besteht;
R'₁ aus der Gruppe ausgewählt ist, die aus H, substituierte oder nicht substituierte nicht zyklische aliphatische Gruppe, substituiertes oder nicht substituiertes Alicyclyl, substituiertes oder nicht substituiertes Heterocyclyl, substituiertes oder nicht substituiertes Heteroarylalkyl, substituiertes oder nicht substituiertes Aryl, substituiertes oder nicht substituiertes Aralkyl und R₅-C(O)- besteht; und
R'₂ aus der Gruppe ausgewählt ist, die aus -NR₃R₄, -OR₃ und -SR₃ besteht; wobei R₃ und R₄ unabhängig aus der Gruppe ausgewählt sind, die aus H, substituierte oder nicht substituierte nicht zyklische aliphatische Gruppe, substituiertes oder nicht substituiertes Alicyclyl, substituiertes oder nicht substituiertes Heterocyclyl, substituiertes oder nicht substituiertes Heteroarylalkyl, substituiertes oder nicht substituiertes Aryl und substituiertes oder nicht substituiertes Aralkyl besteht;
wobei R₅ aus der Gruppe ausgewählt ist, die aus H, substituierte oder nicht substituierte nicht zyklische aliphatische Gruppe, substituiertes oder nicht substituiertes Alicyclyl, substituiertes oder nicht substituiertes Aryl, substituiertes oder nicht substituiertes Aralkyl, substituiertes oder nicht substituiertes Heterocyclyl und substituiertes oder nicht substituiertes Heteroarylalkyl besteht.

7. Verfahren nach Anspruch 6, wobei die Sequenz von benachbarten Aminosäuren, enthalten in der allgemeinen Formel (II), eine Sequenz ist, die aus der Gruppe ausgewählt ist, die aus SEQ ID Nr. 33, SEQ ID Nr. 34, SEQ ID Nr. 35, SEQ ID Nr. 36, SEQ ID Nr. 37 und SEQ ID Nr. 38 besteht.

8. Verfahren nach Anspruch 1, wobei die kosmetische Zusammensetzung ferner eine kosmetisch wirksame Menge von mindestens einem Peptid der allgemeinen Formel (IV):
R"₁-Aₚ-Bᵣ-AA₁-AA₂-AA₃-AA₄-AA₅-AA₆-C₅-Dₜ-R"₂ (IV),
seinen Stereoisomeren, Mischungen davon, und/oder seinen kosmetisch wirksamen Salzen davon enthält, wobei:
AA₁ aus der Gruppe ausgewählt ist, die aus-Asp-, -Glu- und -Pro- besteht;
AA₂ -Asp- ist;
AA₃ aus der Gruppe ausgewählt ist, die aus -Tyr- und -Arg- besteht;
AA₄ aus der Gruppe ausgewählt ist, die aus -Phe- und -Tyr- besteht;
AA₅ aus der Gruppe ausgewählt ist, die aus -Arg- und -Lys- besteht;
AA₆ aus der Gruppe ausgewählt ist, die aus -Leu- und -Met- besteht;
A, B, C und D unabhängig aus der Gruppe ausgewählt sind, die aus natürlichen Aminosäuren und nicht-natürlichen Aminosäuren besteht;
p, r, s und t unabhängig ausgewählt sind und zwischen 0 und 1 betragen;
R"₁ aus der Gruppe ausgewählt ist, die aus H, substituierte oder nicht substituierte nicht zyklische aliphatische Gruppe, substituiertes oder nicht substituiertes Alicyclyl, substituiertes oder nicht substituiertes Heterocyclyl, substituiertes oder nicht substituiertes Heteroarylalkyl, substituiertes oder nicht substituiertes Aryl, substituiertes oder nicht substituiertes Aralkyl und R'₅-C(O)- besteht; und
R"₂ aus der Gruppe ausgewählt ist, die aus -NR'₃R'₄, -OR'₃ und -SR'₃ besteht; wobei R'₃ und R'₄ unabhängig aus der Gruppe ausgewählt sind, die aus H, substituierte oder nicht substituierte nicht zyklische aliphatische Gruppe, substituiertes oder nicht substituiertes Alicyclyl, substituiertes oder nicht substituiertes Heterocyclyl, substituiertes oder nicht substituiertes Heteroarylalkyl, substituiertes oder nicht substituiertes Aryl und substituiertes oder nicht substituiertes Aralkyl besteht;
wobei R'₅ aus der Gruppe ausgewählt ist, die aus H, substituierte oder nicht substituierte nicht zyklische aliphatische Gruppe, substituiertes oder nicht substituiertes Alicyclyl, substituiertes oder nicht substituiertes Aryl, substituiertes oder nicht substituiertes Aralkyl, substituiertes oder nicht substituiertes Heterocyclyl und substituiertes oder nicht substituiertes Heteroarylalkyl besteht.

9. Verfahren nach Anspruch 1, wobei die kosmetische Zusammensetzung auf topischem, transdermalem, enteralem oder parenteralem Weg, durch haftende oder nicht-haftende Pflaster, auf oralem Weg, nasalem Weg oder Inhalationsweg oder durch intradermale, intramuskuläre, intravenöse, intraperitoneale oder subkutane Injektion, Iontophorese, Sonophorese, Elektroporation, mechanischen Druck, osmotischen Druckgradient, okklusive Behandlung, Mikroinjektionen, Druckinjektionen ohne Nadeln oder mikroelektrische Pflaster verabreicht wird.

10. Verfahren nach Anspruch 1, wobei die Peptide der allgemeinen Formel (I) und/oder (II) in ein Zuführungs- oder Retardsystem eingeschlossen sind, ausgewählt aus der Gruppe, die aus Liposomen, gemischten Liposomen, Oleosomen, Niosomen, Minipartikeln, Millipartikeln, Mikropartikeln, Nanopartikeln und festen Lipid-Nanopartikeln, nanostrukturierten Lipidträgern, Schwämmen, Cyclodextrinen, Vesikeln, Mizellen, gemischten Mizellen von Tensiden, gemischten Tensid-Phospholipid-Mizellen, Millikügelchen, Mikrokügelchen, Nanokügelchen, Lipokügelchen, Millikapseln, Mikrokapseln, Nanokapseln, Mikroemulsionen und Nanoemulsionen besteht.

11. Verfahren nach Anspruch 1, wobei die kosmetische Zusammensetzung eine Formulierung aufweist, die aus der Gruppe ausgewählt ist, die aus Cremes, multiplen Emulsionen, wasserfreien Zusammensetzungen, wässrigen Dispersionen, Ölen, Milchen, Balsamen, Schäumen, Lotionen, Gels, Cremegels, wässerig-alkoholischen Lösungen, wässerig-glykolischen Lösungen, Hydrogelen, Linimenten, Sera, Seifen, Shampoos, Konditionierern, Seren, Salben, Schäumen, Pomaden, Pudern, Stäben, Stiften, Sprays und Aerosolen besteht.

12. Verfahren nach Anspruch 1, wobei die kosmetische Zusammensetzung in einem Produkt aufgenommen ist, das aus der Gruppe ausgewählt ist, die aus Kapseln, Injektionsfläschchen, Spritzen, vorgeladenen Spritzen, Under-Eye-Concealern, Make-up-Grundierung, Lotionen zum Entfernen von Make-up, Milchen zum Entfernen von Make-up, Lidschatten, Lippenstiften, Lippenglanz, Lippenschutzmitteln und Pudern besteht.

13. Verfahren nach Anspruch 1, wobei die kosmetische Zusammensetzung in einem Gewebe, einem Vliesstoff, Kleidungsstück oder einer medizinischen Vorrichtung aufgenommen ist.

14. Verfahren nach Anspruch 1, wobei die kosmetische Zusammensetzung ferner eine kosmetisch wirksame Menge von mindestens einem aktiven Stoff umfasst, ausgewählt aus der Gruppe von zyklischen Adenosinmonophosphat-Synthese-stimulierenden Mitteln, Elastase-hemmenden Mitteln, Matrixmetalloproteinase-hemmenden Mitteln, Melaninsynthese-stimulierenden oder - hemmenden Mitteln, Bleichungs- oder Depigmentierungsmitteln, pigmentierungsfördernden Mitteln, Selbstbräunungsmitteln, Alterungsschutzmitteln, NO-Synthase-hemmenden Mitteln, 5α-Reduktasehemmenden Mitteln, Lysyl- und/oder Prolyl-Hydroxylase-hemmenden Mitteln, Antioxidationsmitteln, Radikalfängern und/oder Mitteln gegen Luftverschmutzung, Fängern für reaktive Carbonylarten, Mitteln gegen Glykation, Antihistaminmitteln, antiviralen Mitteln, antiparasitischen Mitteln, Emulgatoren, Erweichungsmitteln, organischen Lösungsmitteln, flüssigen Treibmitteln, Haut- und/oder Haar-Konditionierern, Feuchthaltemitteln, Substanzen, die die Feuchtigkeit zurückhalten, Alpha-Hydroxysäuren, Beta-Hydroxysäuren, Feuchtigkeitsspendern, epidermalen hydrolytischen Enzymen, Vitaminen, Pigmenten oder Färbemitteln, Farbstoffen, gelierenden Polymeren, Verdickungsmitteln, Tensiden, Weichmachern, Antifaltenmitteln, Mitteln, die in der Lage sind, die Tränensäcke zu reduzieren oder zu behandeln, Abblätterungsmitteln, antimikrobiellen Mitteln, antifungalen Mitteln, fungistatischen Mitteln, bakteriziden Mitteln, bakteriostatischen Mitteln, Mitteln, die die Synthese von dermalen oder epidermalen Makromolekülen stimulieren und/oder in der Lage sind, deren Zersetzung zu hemmen oder zu verhindern, die Kollagen-Synthese stimulierenden Mitteln, die Elastin-Synthese stimulierenden Mitteln, die Decorin-Synthese stimulierenden Mitteln, die Laminin-Synthese stimulierenden Mitteln, die Defensin-Synthese stimulierenden Mitteln, die Chaperon-Synthese stimulierenden Mitteln, die Aquaporin-Synthese stimulierenden Mitteln, die Hyaluronsäure-Synthese stimulierenden Mitteln, die Fibronectin-Synthese stimulierenden Mitteln, die Sirtuin-Synthese stimulierenden Mitteln, Mitteln, die die Synthese von Lipiden und Komponenten des Stratum corneum stimulieren, Mitteln, die die Synthese von Ceramiden stimulieren, Mitteln, die die Kollagen-Zersetzung hemmen, Mitteln, die die Elastin-Zersetzung hemmen, Mitteln, die die Serin-Proteasen, wie zum Beispiel Cathepsin G, hemmen, Mitteln, die die Fibroplast-Proliferation stimulieren, Mitteln, die die Keratinozyt-Proliferation stimulieren, Mitteln, die die Adipozyt-Proliferation stimulieren, Mitteln, die die Melanozyt-Proliferation stimulieren, Mitteln, die die Keratinozyt-Differenzierung stimulieren, Mitteln, die die Adipozyt-Differenzierung stimulieren, Mitteln, die die Acetylcholinesterase hemmen, Hautentspannungsmitteln, Mitteln, die die Acetylcholin-Rezeptor-Aggregation hemmen, Mitteln, die die Muskelkontraktion hemmen, die Glycosaminoglykan-Synthese stimulierenden Mitteln, Anti-Hyperkeratose-Mitteln, komedolytischen Mitteln, Antipsoriasismitteln, DNA-Reparatur-Mitteln, DNA-Schutzmitteln, Stabilisatoren, Anti-Juckreiz-Mitteln, Mitteln für die Behandlung und/oder Pflege von empfindlicher Haut, Straffungsmitteln, Mitteln gegen Dehnungsstreifen, Bindemitteln, Mitteln, die die Talgproduktion regulieren, lipolytischen Mitteln oder Mitteln, die die Lipolyse stimulieren, Anti-Cellulite-Mitteln, Antiperspirant-Mitteln, Mitteln, die die Heilung stimulieren, Coadjuvans-Heilmitteln, Mitteln, die die Reeptithelisierung stimulieren, Coadjuvans-Reepithelisierungsmitteln, Cytokin-Wachstumsfaktoren, Beruhigungsmitteln, entzündungshemmenden Mitteln, Betäubungsmitteln, Mitteln, die auf Kapillarzirkulation und/oder Mikrozirkulation wirken, Mitteln, die die Angiogenese stimulieren, Mitteln, die die Gefäßdurchlässigkeit hemmen, venotonischen Mitteln, Mitteln, die auf den Zellmetabolismus wirken, Mitteln zum Verbessern der dermal-epidermalen Verbindung, Mitteln zur Anregung des Haarwachstums, den Haarwachstum hemmenden oder verzögernden Mitteln, Konservierungsstoffen, Duftstoffen, chelatisierenden Mitteln, pflanzlichen Extrakten, ätherischen Ölen, Meeresextrakten, Mitteln, die anhand eines Biofermentationsprozesses erhalten werden, Mineralsalzen, Zellextrakten und Sonnenschutzmitteln, organischen oder mineralen Lichtschutzmitteln, die gegen ultraviolette A- und/oder B-Strahlen wirken und/oder Mischungen davon.

15. Kit zur Anti-Aging-Behandlung nach Anspruch 1, umfassend:
a. Botulinumtoxin,
b. und mindestens eine kosmetische Zusammensetzung, die eine kosmetisch wirksame Menge von mindestens einem Peptid, das aus der Gruppe ausgewählt ist, die aus SEQ ID Nr. 11, SEQ ID Nr. 4 oder einer Sequenz von 7 bis 12 benachbarten Aminosäuren, die in SEQ ID NR. 4 enthalten sind, besteht, wobei die Sequenz die Aminosäurensequenz von SEQ ID NR. 11 umfasst, und gemäß der allgemeinen Formel (I):
R₁-AA-R₂ (I),
seinen Stereoisomeren, Mischungen davon und/oder seinen kosmetisch verträglichen Salzen davon, wobei AA eine Sequenz ist, die aus der Gruppe ausgewählt ist, die aus SEQ ID Nr. 11, SEQ ID Nr. 4 oder einer Sequenz von 7 bis 12 benachbarten Aminosäuren, die in SEQ ID NR. 4 enthalten sind, besteht, wobei die Sequenz die Aminosäurensequenz von SEQ ID Nr. 11 umfasst;
wobei:
R₁ aus der Gruppe ausgewählt ist, die aus H, substituierte oder nicht substituierte nicht zyklische aliphatische Gruppe, substituiertes oder nicht substituiertes Alicyclyl, substituiertes oder nicht substituiertes Heterocyclyl, substituiertes oder nicht substituiertes Heteroarylalkyl, substituiertes oder nicht substituiertes Aryl, substituiertes oder nicht substituiertes Aralkyl und R₅-C(O)- besteht; und
R₂ aus der Gruppe ausgewählt ist, die aus -NR₃R₄, -OR₃ und -SR₃ besteht; wobei R₃ und R₄ unabhängig aus der Gruppe ausgewählt sind, die aus H, substituierte oder nicht substituierte nicht zyklische aliphatische Gruppe, substituiertes oder nicht substituiertes Alicyclyl, substituiertes oder nicht substituiertes Heterocyclyl, substituiertes oder nicht substituiertes Heteroarylalkyl, substituiertes oder nicht substituiertes Aryl und substituiertes oder nicht substituiertes Aralkyl besteht;
wobei R₅ aus der Gruppe ausgewählt ist, die aus H, substituierte oder nicht substituierte nicht zyklische aliphatische Gruppe, substituiertes oder nicht substituiertes Alicyclyl, substituiertes oder nicht substituiertes Aryl, substituiertes oder nicht substituiertes Aralkyl, substituiertes oder nicht substituiertes Heterocyclyl und substituiertes oder nicht substituiertes Heteroarylalkyl besteht;
und mindestens einen kosmetisch verträglichen Excipienten oder Adjuvans umfasst.

16. Kit nach Anspruch 15, wobei die kosmetische Zusammensetzung ferner eine kosmetisch wirksame Menge von mindestens einem von Enkephalin abgeleiteten Peptid der allgemeinen Formel (II): 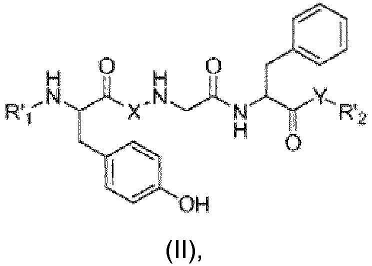 seinen Stereoisomeren, Mischungen davon und/oder seinen kosmetisch verträglichen Salzen davon, enthält,
wobei:
X und Y unabhängig aus der Gruppe ausgewählt sind, die aus natürlichen Aminosäuren und nicht-natürlichen Aminosäuren besteht;
R'₁ aus der Gruppe ausgewählt ist, die aus H, substituierte oder nicht substituierte nicht zyklische aliphatische Gruppe, substituiertes oder nicht substituiertes Alicyclyl, substituiertes oder nicht substituiertes Heterocyclyl, substituiertes oder nicht substituiertes Heteroarylalkyl, substituiertes oder nicht substituiertes Aryl, substituiertes oder nicht substituiertes Aralkyl und R₅-C(O)- besteht; und
R'₂ aus der Gruppe ausgewählt ist, die aus -NR₃R₄, -OR₃ und -SR₃ besteht, wobei R₃ und R₄ unabhängig aus der Gruppe ausgewählt sind, die aus H, substituierte oder nicht substituierte nicht zyklische aliphatische Gruppe, substituiertes oder nicht substituiertes Alicyclyl, substituiertes oder nicht substituiertes Heterocyclyl, substituiertes oder nicht substituiertes Heteroarylalkyl, substituiertes oder nicht substituiertes Aryl und substituiertes oder nicht substituiertes Aralkyl besteht;
wobei R₅ aus der Gruppe ausgewählt ist, die aus H, substituierte oder nicht substituierte nicht zyklische aliphatische Gruppe, substituierte oder nicht substituiertes Alicyclyl, substituiertes oder nicht substituiertes Aryl, substituiertes oder nicht substituiertes Aralkyl, substituiertes oder nicht substituiertes Heterocyclyl und substituiertes oder nicht substituiertes Heteroarylalkyl besteht,
und/oder eine kosmetisch wirksame Menge von mindestens einem Peptid der allgemeinen Formel (IV):
R"₁-Aₚ-Bᵣ-AA₁-AA₂-AA₃-AA₄-AA₅-AA₆-Cₛ-Dt-R"₂ (IV),
seinen Stereoisomeren, Mischungen davon und/oder seinen kosmetisch verträglichen Salzen davon, wobei:
AA₁ aus der Gruppe ausgewählt ist, die aus -Asp-, -Glu- und -Probesteht;
AA₂ -Asp- ist;
AA₃ aus der Gruppe ausgewählt ist, die aus -Tyr- und -Arg- besteht;
AA₄ aus der Gruppe ausgewählt ist, die aus -Phe- und -Tyr- besteht;
AA₅ aus der Gruppe ausgewählt ist, die aus -Leu- und -Met- besteht;
A, B, C und D unabhängig aus der Gruppe ausgewählt sind, die aus natürlichen Aminosäuren und nicht-natürlichen Aminosäuren besteht;
p, r, s und t unabhängig ausgewählt sind und zwischen 0 und 1 betragen; R"₁ aus der Gruppe ausgewählt ist, die aus H, substituierte oder nicht substituierte nicht zyklische aliphatische Gruppe, substituiertes oder nicht subsituiertes Alicyclyl, substituiertes oder nicht substituiertes Heterocyclyl, substituiertes oder nicht substituiertes Heteroarylalkyl, substituiertes oder nicht substituiertes Aryl, substituiertes oder nicht substituiertes Aralkyl und R'₅-C(O)- besteht; und
R"₂ aus der Gruppe ausgewählt ist, die aus -NR'₃R₄, -OR'₃ und -SR'₃ besteht; wobei R'₃ und R'₄ unabhängig aus der Gruppe ausgewählt sind, die aus H, substituierte oder nicht substituierte nicht zyklische aliphatische Gruppe, substituiertes oder nicht substituiertes Alicyclyl, substituiertes oder nicht substituiertes Heterocyclyl, substituiertes oder nicht substituiertes Heteroarylalkyl, substituiertes oder nicht substituiertes Aryl und substituiertes oder nicht substituiertes Aralkyl besteht;
wobei R'₅ aus der Gruppe ausgewählt ist, die aus H, substituierte oder nicht substituierte nicht zyklische aliphatische Gruppe, substituiertes oder nicht substituiertes Alicyclyl, substituiertes oder nicht substituiertes Aryl, substituiertes oder nicht substituiertes Aralkyl, substituiertes oder nicht substituiertes Heterocyclyl und substituiertes oder nicht substituiertes Heteroarylalkyl besteht.

## Revendications

1. Procédé non thérapeutique pour un traitement antivieillissement pour la peau qui prolonge dans le temps les effets du traitement avec des injections de toxine botulique, le procédé comprenant :
a. L'administration d'une dose efficace de toxine botulique à une zone de la peau du visage et/ou du cou,
b. et l'administration, d'une fois par semaine à dix fois par jour, d'une composition cosmétique comprenant une quantité cosmétiquement efficace d'au moins un peptide choisi dans le groupe consistant en SEQ ID No. 11, SEQ ID No. 4, ou une séquence de 7 à 12 acides aminés adjacents contenue dans SEQ ID No. 4, dans laquelle ladite séquence comprend la séquence d'acides aminés de SEQ ID No. 11, et selon la formule générale (I) :
R₁-AA-R₂ (I)
ses stéréoisomères, leurs mélanges, et / ou leurs sels cosmétiquement acceptables, dans laquelle AA est une séquence choisie dans le groupe consistant en SEQ ID No. 11, SEQ ID No. 4, ou une séquence de 7 à 12 acides aminés adjacents contenue dans SEQ ID No. 4, dans laquelle ladite séquence comprend la séquence d'acides aminés de SEQ ID No.11 ; dans laquelle :
R₁ est choisi dans le groupe consistant en H, un groupe aliphatique non-cyclique substitué ou non substitué, un alicyclyle substitué ou non substitué, un hétérocyclyle substitué ou non substitué, un hétéroarylalkyle substitué ou non substitué, un aryle substitué ou non substitué, un aralkyle substitué ou non substitué et R₅-C(O)- ; et R₂ est choisi dans le groupe consistant en - NR₃R₄, -OR₃ et -SR₃ ; où R₃ et R₄ sont indépendamment choisis dans le groupe consistant en H, un groupe aliphatique non-cyclique substitué ou non substitué, un alicyclyle substitué ou non substitué, un hétérocyclyle substitué ou non substitué, un hétéroarylalkyle substitué ou non substitué, un aryle substitué ou non substitué et un aralkyle substitué ou non substitué ;
dans lequel R₅ est choisi dans le groupe consistant en H, un groupe aliphatique non-cyclique substitué ou non substitué, un alicyclyle substitué ou non substitué, un aryle substitué ou non substitué, un aralkyle substitué ou non substitué, un hétérocyclyle substitué ou non substitué et un hétéroarylalkyle substitué ou non substitué ;
et au moins un excipient ou un adjuvant cosmétiquement acceptable.

2. Procédé selon la revendication 1, dans lequel le traitement antivieillissement pour la peau est un traitement pour réduire ou éliminer les rides de la peau du visage et/ou du cou.

3. Procédé selon la revendication 1, dans lequel R₁ est choisi dans le groupe consistant en H, un polymère de formule générale (III) 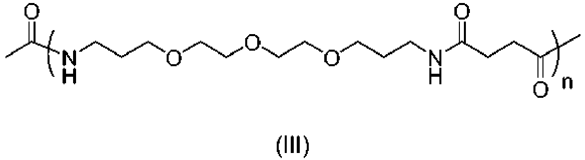 dans laquelle n varie entre 1 à 100, et R₅-CO-, dans lequel R₅ est choisi dans le groupe consistant en un radical alkyle en C1-C24 substitué ou non substitué, un radical alkényle en C₂-C₂₄ substitué ou non substitué, un radical alkinyle en C₂-C₂₄ substitué ou non substitué, un radical cycloalkyle en C₃-C₂₄ substitué ou non substitué, un radical cycloalkényle en C₅-C₂₄ substitué ou non substitué, un radical cycloalkinyle en C₈-C₂₄ substitué ou non substitué, un radical aryle en C₆-C₃₀ substitué ou non substitué, un radical aralkyle en C₇-C₂₄ substitué ou non substitué, un radical hétérocyclyle substitué ou non substitué ayant 3-10 chaînons, un radical hétéroaralkyle substitué ou non substitué ayant 2 à 24 atomes de carbone et ayant 1 à 3 atomes autres que le carbone dans lequel la chaîne alkyle a 1 à 6 atomes de carbone.

4. Procédé selon la revendication 1, dans lequel R₃ et R₄ sont indépendamment choisis dans le groupe qui consiste en H, un alkyle en C₁-C₂₄ substitué ou non substitué, un alkényle en C₂-C₂₄ substitué ou non substitué, un alkinyle en C₂-C₂₄ substitué ou non substitué, un cycloalkyle en C₃-C₂₄ substitué ou non substitué, un cycloalkényle en C₅-C₂₄ substitué ou non substitué, un cycloalkinyle en C₈-C₂₄ substitué ou non substitué, un aryle en C₆-C₃₀ substitué ou non substitué, un aralkyle en C₇-C₂₄ substitué ou non substitué, un radical hétérocyclyle substitué ou non substitué ayant 3-10 chaînons, et un groupe hétéroarylalkyle substitué ou non substitué ayant 2 à 24 atomes de carbone et ayant 1 à 3 atomes autres que le carbone dans lequel la chaîne alkyle a 1 à 6 atomes de carbone et un polymère de formule générale (III) dans laquelle n varie entre 1 à 100.

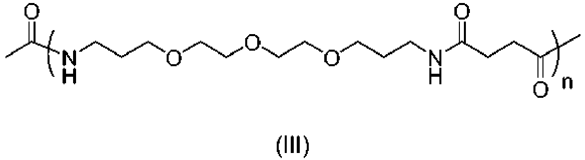

5. Procédé selon la revendication 1, dans lequel AA est une séquence d'acides aminés choisie dans le groupe consistant en SEQ ID No. 4, SEQ ID No.8, SEQ ID No.11, SEQ ID No.14, SEQ ID No.15, SEQ ID No 16, SEQ ID No.17, SEQ ID No.18, SEQ ID No.19, SEQ ID No.20, SEQ ID No.21, SEQ ID No.22, SEQ ID No.23, SEQ ID No.24, SEQ ID No.25 et SEQ ID No.26.

6. Procédé selon la revendication 1, dans lequel la composition cosmétique contient en outre une quantité cosmétiquement efficace d'au moins un peptide dérivé de l'enképhaline de formule générale (II) : 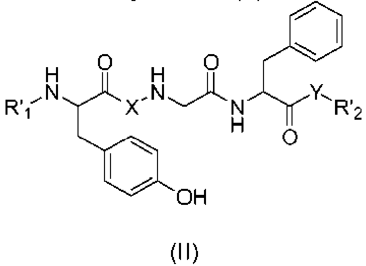 ses stéréoisomères, leurs mélanges, et / ou leurs sels cosmétiquement acceptables,
X et Y sont choisis indépendamment dans le groupe constitué par les acides aminés naturels et les acides aminés non naturels ;
R'₁ est choisi dans le groupe consistant en H, un groupe aliphatique non-cyclique substitué ou non substitué, un alicyclyle substitué ou non substitué, un hétérocyclyle substitué ou non substitué, un hétéroarylalkyle substitué ou non substitué, un aryle substitué ou non substitué, un aralkyle substitué ou non substitué et R₅-C(O)- ; et R'₂ est choisi dans le groupe consistant en - NR₃R₄, -OR₃ et -SR₃ ; où R₃ et R₄ sont indépendamment choisis dans le groupe consistant en H, un groupe aliphatique non-cyclique substitué ou non substitué, un alicyclyle substitué ou non substitué, un hétérocyclyle substitué ou non substitué, un hétéroarylalkyle substitué ou non substitué, un aryle substitué ou non substitué et un aralkyle substitué ou non substitué ;
dans lequel R₅ est choisi dans le groupe consistant en H, un groupe aliphatique non-cyclique substitué ou non substitué, un alicyclyle substitué ou non substitué,
un aryle substitué ou non substitué, un aralkyle substitué ou non substitué, un hétérocyclyle substitué ou non substitué et un hétéroarylalkyle substitué ou non substitué.

7. Procédé selon la revendication 6, dans lequel la séquence d'acides aminés adjacents contenus dans la formule générale (II) est une séquence choisie dans le groupe consistant en SEQ ID No. 33, SEQ ID No.34, SEQ ID No.35, SEQ ID No.36, SEQ ID No.37 et SEQ ID No.38.

8. Procédé selon la revendication 1, dans lequel la composition cosmétique contient en outre une quantité cosmétiquement efficace d'au moins un peptide de formule générale (IV) :
R"₁-Aₚ-Bᵣ-AA₁-AA₂-AA₃-AA₄-AA₅-AA₆-Cₛ-DₜR"₂ (IV)
ses stéréoisomères, leurs mélanges, et / ou leurs sels cosmétiquement acceptables, dans laquelle :
AA₁ est choisi dans le groupe consistant en -Asp-, -Glu- et -Pro- ;
AA₂ est -Asp- ;
AA₃ est choisi dans le groupe consistant en -Tyr- et -Arg- ;
AA₄ est choisi dans le groupe consistant en -Phe- et -Tyr- ;
AA₅ est choisi dans le groupe consistant en -Arg- et -Lys- ;
AA₆ est choisi dans le groupe consistant en -Leu- et -Met- ;
A, B, C et D sont choisis indépendamment dans le groupe constitué par les acides aminés naturels et les acides aminés non naturels ;
p, r, s et t sont choisis indépendamment et varient entre 0 et 1 ;
R"₁ est choisi dans le groupe consistant en H, un groupe aliphatique non-cyclique substitué ou non substitué, un alicyclyle substitué ou non substitué, un hétérocyclyle substitué ou non substitué, un hétéroarylalkyle substitué ou non substitué, un aryle substitué ou non substitué, un aralkyle substitué ou non substitué et R'_{'5}-C(O)- , et
R"₂ est choisi dans le groupe consistant en -NR'₃R₄, -OR'₃ et -SR'₃ ; où R'₃ et R'₄ sont indépendamment choisis dans le groupe consistant en H, un groupe aliphatique non-cyclique substitué ou non substitué,
un alicyclyle substitué ou non substitué, un hétérocyclyle substitué ou non substitué, un hétéroarylalkyle substitué ou non substitué, un aryle substitué ou non substitué et un aralkyle substitué ou non substitué ;
dans lequel R'₅ est choisi dans le groupe consistant en H, un groupe aliphatique non-cyclique substitué ou non substitué, un alicyclyle substitué ou non substitué, un aryle substitué ou non substitué, un aralkyle substitué ou non substitué, un hétérocyclyle substitué ou non substitué et un hétéroarylalkyle substitué ou non substitué.

9. Procédé selon la revendication 1, dans lequel la composition cosmétique est administrée par timbres topiques, transdermiques, voie entérale ou parentérale, adhésives ou non adhésives, voie orale, nasale ou inhalatoire ou par injection intradermique, intramusculaire, intraveineuse, intrapéritonéale ou sous-cutanée, iontophorèse, sonophorèse, électroporation, pression mécanique, gradient de pression osmotique, traitement occlusif, micro-injections, injections sous pression sans aiguilles ou timbres micro-électriques.

10. Procédé selon la revendication 1, dans lequel les peptides de formule générale (I) et/ou (II) sont incorporés dans un système de délivrance ou de libération prolongée choisi dans le groupe consistant en les liposomes, les liposomes mélangés, les oléosomes, les niosomes, les miniparticules, les milliparticules, les microparticules, les nanoparticules et les nanoparticules lipidiques solides, les porteurs de lipides nanostructurés, les éponges, les cyclodextrines, les vésicules, les micelles, les micelles mixtes de tensioactifs, les micelles mixtes tensioactif-phospholipides, les millisphères, les microsphères, les nanosphères, les liposphères, les millicapsules, les microcapsules, les nanocapsules, les microémulsions et les nanoémulsions.

11. Procédé selon la revendication 1, dans lequel la composition cosmétique présente une formulation choisie dans le groupe consistant en crèmes, émulsions multiples, compositions anhydres, dispersions aqueuses, huiles, laits, baumes, mousses, lotions, gels, gels crèmes, solutions hydroalcooliques, solutions hydroglycoliques, hydrogels, liniments, sérums, savons, shampooings, après-shampoings, sérums, onguents, mousses, pommades, poudres, barres, crayons, sprays et aérosols.

12. Procédé selon la revendication 1, dans lequel la composition cosmétique se trouve incorporée dans un produit choisi dans le groupe consistant en des capsules, des flacons, des seringues, des seringues préchargées, des anti-cernes sous les yeux, des fonds de teint de maquillage, des lotions démaquillantes, des laits démaquillants, des ombres à paupières, des rouges à lèvres, des brillants à lèvres, des protecteurs pour les lèvres et des poudres.

13. Procédé selon la revendication 1, dans lequel la composition cosmétique est incorporée dans un tissu, un tissu non tissé, un vêtement ou un dispositif médical.

14. Procédé selon la revendication 1, dans lequel la composition cosmétique comprend en outre une quantité cosmétiquement efficace d'au moins un agent actif choisi dans le groupe des agents stimulateurs de la synthèse de l'adénosine monophosphate cyclique, des agents inhibiteurs de l'élastase, des agents inhibiteurs des métalloprotéinases matricielles, des agents stimulateurs ou inhibiteurs de la synthèse de la mélanine, des agents blanchissants ou dépigmentants, des agents de propigmentations, des agents autobronzants, des agents antivieillissement, des agents inhibiteurs de l'oxyde nitrique synthase, des agents inhibiteurs de 5-α réductase, des agents inhibiteurs de lysine et/ou prolyl hydroxylase, des antioxydants, des piégeurs de radicaux libres et/ou agents anti-pollution, des piégeurs d'espèces carbonylées réactifs, des agents anti-glycation, des agents antihistaminiques, des agents antiviraux, des agents antiparasitaires, des émulsifiants, des émollients, des solvants organiques, des propulseurs liquides, des conditionneurs capillaires ou cutanés, des humectants, des substances qui retiennent l'humidité, des acides alpha-hydroxylés, des acides bêta-hydroxylés, des crèmes hydratantes, des enzymes hydrolytiques épidermiques, des vitamines, des pigments ou des colorants, des teintures, des polymères gélifiants, des épaississants, des tensioactifs, des agents adoucissants, des agents anti-rides, des agents capables de réduire ou de traiter des poches sous les yeux, des agents exfoliants, des agents antimicrobiens, des agents antifongiques, des agents fongistatiques, des agents bactéricides, des agents bactériostatiques, des agents simulant la synthèse des macromolécules dermiques ou épidermiques et/ou capables d'inhiber ou de prévenir leur dégradation, des agents stimulateurs de la synthèse de collagène, des agents stimulateurs de la synthèse d'élastine, des agents stimulateurs de la synthèse de décorine, des agents stimulateurs de la synthèse de laminine, des agents stimulateurs de la synthèse de défensine, des agents stimulateurs de la synthèse de chaperon, des agents stimulateurs de la synthèse d'aquaporine, des agents stimulateurs de la synthèse d'acide hyaluronique, des agents stimulateurs de la synthèse de fibronectine, des agents stimulateurs de la synthèse de sirtuine, des agents stimulateurs de la synthèse des lipides et de composants de la couche cornée, des agents stimulateurs de la synthèse des céramides, des agents qui inhibent la dégradation du collagène, des agents qui inhibent la dégradation d'élastine, des agents qui inhibent des sérine-protéases telles que la cathepsine G, des agents qui stimulent la prolifération des fibroblastes, des agents qui stimulent la prolifération des kératinocytes, des agents qui stimulent la prolifération d'adipocytes, des agents qui stimulent la prolifération des mélanocytes, des agents qui stimulent la différenciation des kératinocytes, des agents qui stimulent la différenciation d'adipocytes, des agents qui inhibent l'acétylcholinestérase, des agents relaxants pour la peau, des agents qui inhibent l'agrégation des récepteurs de l'acétylcholine, des agents qui inhibent la contraction musculaire, des agents qui stimulent la synthèse de glycosaminoglycane, des agents antihyperkératoses, des agents comédolytiques, des agents anti-psoriasis, des agents de réparation de l'ADN, des agents de protection de l'ADN, des stabilisants, des agents anti-démangeaisons, des agents en vue du traitement et/ou des soins des peaux sensibles, des agents raffermissants, des agents anti-vergetures, des agents liants, des agents régulant la production de sébum, des agents lipolytiques ou des agents qui stimulent la lipolyse, des agents anticellulites, des agents antisudorifiques, des agents qui stimulent la guérison, des agents co-adjuvants de la guérison, des agents qui stimulent la réépithélisation, des agents co-adjuvants de la réépithélisation, des facteurs de croissance de cytokines, des agents calmants, des agents anti-inflammatoires, des agents d'anesthésie, des agents agissant sur la circulation capillaire et/ou la microcirculation, des agents qui stimulent l'angiogenèse, des agents qui inhibent la perméabilité vasculaire, des agents veinotoniques, des agents agissant sur le métabolisme cellulaire, des agents qui améliorent la jonction dermo-épidermique, des agents favorisant la croissance capillaire, des agents inhibiteurs de croissance capillaire ou des agents retardateurs, des préservatifs, des parfums, des agents chélateurs, des extraits végétaux, des huiles essentielles, des extraits marins, des agents obtenus du processus de biofermentation, des sels minéraux, des extraits cellulaires et des crèmes solaires, des agents photoprotecteurs organiques ou minéraux actifs contre les rayons ultraviolets A et/ou B, ou des combinaisons de ceux-ci.

15. Kit pour le traitement antivieillissement pour la peau selon la revendication 1, comprenant :
a. toxine botulique,
b. et au moins une composition cosmétique comprenant une quantité cosmétiquement efficace d'au moins un peptide choisi dans le groupe consistant en SEQ ID No. 11, SEQ ID No. 4, ou une séquence de 7 à 12 acides aminés adjacents contenue dans SEQ ID No. 4, dans laquelle ladite séquence comprend la séquence d'acides aminés de SEQ ID No. 11, et selon la formule générale (I) :
R₁-AA-R₂ (I)
ses stéréoisomères, leurs mélanges, et / ou leurs sels cosmétiquement acceptables, dans laquelle AA est une séquence choisie dans le groupe consistant en SEQ ID No. 11, SEQ ID No. 4, ou une séquence de 7 à 12 acides aminés adjacents contenue dans SEQ ID No. 4, dans laquelle ladite séquence comprend la séquence d'acides aminés de SEQ ID No.11 ;
dans laquelle :
R₁ est choisi dans le groupe consistant en H, un groupe aliphatique non-cyclique substitué ou non substitué, un alicyclyle substitué ou non substitué, un hétérocyclyle substitué ou non substitué, un hétéroarylalkyle substitué ou non substitué, un aryle substitué ou non substitué, un aralkyle substitué ou non substitué et R₅-C(O)- ; et
R₂ est choisi dans le groupe consistant en -NR₃R₄, -OR₃ et -SR₃ ; où R₃ et R₄ sont indépendamment choisis dans le groupe consistant en H, un groupe aliphatique non-cyclique substitué ou non substitué, un alicyclyle substitué ou
non substitué, un hétérocyclyle substitué ou non substitué, un hétéroarylalkyle substitué ou non substitué, un aryle substitué ou non substitué et un aralkyle substitué ou non substitué ;
dans lequel R₅ est choisi dans le groupe consistant en H, un groupe aliphatique non-cyclique substitué ou non substitué, un alicyclyle substitué ou non substitué, un aryle substitué ou non substitué, un aralkyle substitué ou non substitué, un hétérocyclyle substitué ou non substitué et un hétéroarylalkyle substitué ou non substitué ;
et au moins un excipient ou un adjuvant cosmétiquement acceptable.

16. Kit selon la revendication 15, dans lequel la composition cosmétique contient en outre une quantité cosmétiquement efficace d'au moins un peptide dérivé de l'enképhaline de formule générale (II) : 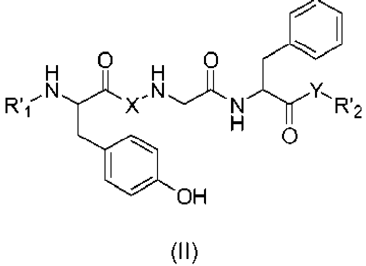 ses stéréoisomères, leurs mélanges, et / ou leurs sels cosmétiquement acceptables,
dans laquelle
X et Y sont choisis indépendamment dans le groupe constitué par les acides aminés naturels et les acides aminés non naturels ;
R'₁ est choisi dans le groupe consistant en H, un groupe aliphatique non-cyclique substitué ou non substitué, un alicyclyle substitué ou non substitué, un hétérocyclyle substitué ou non substitué, un hétéroarylalkyle substitué ou non substitué, un aryle substitué ou non substitué, un aralkyle substitué ou non substitué et R₅-C(O)- ; et
R'₂ est choisi dans le groupe consistant en -NR₃R₄, -OR₃ et -SR₃ ; où R₃ et R₄ sont indépendamment choisis dans le groupe consistant en H, un groupe aliphatique non-cyclique substitué ou non substitué, un alicyclyle substitué ou non substitué, un hétérocyclyle substitué ou non substitué, un hétéroarylalkyle substitué ou non substitué, un aryle substitué ou non substitué et un aralkyle substitué ou non substitué ;
dans lequel R₅ est choisi dans le groupe consistant en H, un groupe aliphatique non-cyclique substitué ou non substitué, un alicyclyle substitué ou non substitué, un aryle substitué ou non substitué, un aralkyle substitué ou non substitué, un hétérocyclyle substitué ou non substitué et un hétéroarylalkyle substitué ou non substitué,
et/ou une quantité cosmétiquement efficace d'au moins un peptide de formule générale (IV) :
R"₁-Aₚ-Bᵣ-AA₁-AA₂-AA₃-AA₄-AA₅-AA₆-Cₛ-Dₜ-R"₂ (IV)
ses stéréoisomères, leurs mélanges, et / ou leurs sels cosmétiquement acceptables, dans laquelle :
AA₁ est choisi dans le groupe consistant en -Asp-, -Glu- et -Pro- ;
AA₂ est -Asp- ;
AA₃ est choisi dans le groupe consistant en -Tyr- et -Arg- ;
AA₄ est choisi dans le groupe consistant en -Phe- et -Tyr- ;
AA₅ est choisi dans le groupe consistant en -Arg- et -Lys- ;
AA₆ est choisi dans le groupe consistant en -Leu- et -Met- ;
A, B, C et D sont choisis indépendamment dans le groupe constitué par les acides aminés naturels et les acides aminés non naturels ;
p, r, s et t sont choisis indépendamment et varient entre 0 et 1 ;
R"₁ est choisi dans le groupe consistant en H, un groupe aliphatique non-cyclique substitué ou non substitué, un alicyclyle substitué ou non substitué, un hétérocyclyle substitué ou non substitué, un hétéroarylalkyle substitué ou non substitué, un aryle substitué ou non substitué, un aralkyle substitué ou non substitué et R'_{'5}-C(O)- ; et
R"₂ est choisi dans le groupe consistant en -N'_{'3}R'4, -OR'₃ et -SR'₃ ; où R'₃ et R'₄ sont indépendamment choisis dans le groupe consistant en H, un groupe aliphatique non-cyclique substitué ou non substitué, un alicyclyle substitué ou non substitué, un hétérocyclyle substitué ou non substitué, un hétéroarylalkyle substitué ou non substitué, un aryle substitué ou non substitué et un aralkyle substitué ou non substitué ;
dans lequel R'₅ est choisi dans le groupe consistant en H, un groupe aliphatique non-cyclique substitué ou non substitué, un alicyclyle substitué ou non substitué, un aryle substitué ou non substitué, un aralkyle substitué ou non substitué, un hétérocyclyle substitué ou non substitué et un hétéroarylalkyle substitué ou non substitué.
